# EUROPEAN PATENT SPECIFICATION

(11) **EP 1 150 991 B1**
(45) Date of publication and mention of the grant of the patent: **07.04.2004**
(21) Application number: 00905833.0
(22) Date of filing: 31.01.2000
(51) Int. Cl.: C07H 19/16

(54) **COMPOSITIONS FOR TREATING INFLAMMATORY RESPONSE**
ZUSAMMENSETZUNGEN ZUR BEHANDLUNG VON ENTZÜNDUNGSREAKTIONEN
COMPOSITIONS PERMETTANT DE TRAITER UNE REPONSE INFLAMMATOIRE

(30) Priority: 01.02.1999 US 118029 P; 12.03.1999 US 124316 P; 10.05.1999 US 133374 P; 24.05.1999 US 135573 P; 15.06.1999 US 333387; 30.08.1999 US 151412 P
(43) Date of publication of application: 07.11.2001
(73) Proprietor: University of Virginia Patent Foundation, Charlottesville, VA 22903-2442 (US)
(72) Inventor: LINDEN, Joel, M., Charlottesville, VA 22903 (US); SULLIVAN, Gail, W., Charlottesville, VA 22903 (US); SAREMBOCK, Ian, J., Charlottesville, VA 22903 (US); MACDONALD, Timothy, Charlottesville, VA 22903 (US); OKUSA, Mark, Charlottesville, VA 22901 (US); KRON, Irving, L., Charlottesville, VA 22901 (US); SCHELD, W., Michael, Earlysville, VA 22936 (US)
(74) Representative: Lucas, Brian Ronald
(86) International application number: PCT/US2000/002324
(87) International publication number: WO 2000/044763

(56) References cited:
- US-A- 5 593 975
- BARALDI, PIER GIOVANNI ET AL: "Synthesis and Biological Activity of a New Series of N6-Arylcarbamoyl, 2-(Ar)alkynyl-N6-arylcarbamoyl, and N6-Carboxamido Derivatives of Adenosine-5'-N-ethyluronamide as A1 and A3 Adenosine Receptor Agonists" J. MED. CHEM. (1998), 41(17), 3174-3185 , XP002149470
- MAGER, PETER P.: "Neural network approaches applied to selective A2a adenosine receptor agonists" MED. CHEM. RES. (1998), 8(6), 277-290 , XP000952528
- OKUSA, MARK D. ET AL: "Selective A2A adenosine receptor activation reduces ischemia-reperfusion injury in rat kidney" AM. J. PHYSIOL. (1999), 277(3, PT. 2), F404-F412 , XP000952524

## Description

### Field of the Invention

The present invention relates to methods and compositions for preventing tissue injury, i.e., due to inflammatory activity.

### Background of the Invention

The inflammatory response serves the purpose of eliminating harmful agents from the body. There is a wide range of pathogenic insults that can initiate an inflammatory response including infection, allergens, autoimmune stimuli, immune response to transplanted tissue, noxious chemicals, and toxins, ischemia/reperfusion, hypoxia. mechanical and thermal trauma. Inflammation normally is a very localized action which serves in expulsion, attenuation by dilution, and isolation of the damaging agent and injured tissue. The body's response becomes an agent of disease when it results in inappropriate injury to host tissues in the process of eliminating the targeted agent, or responding to a traumatic insult.

As examples, inflammation is a component of pathogenesis in several vsscular diseases or injuries. Examples include: ischemia/reperfusion injury (N. G. Frangogiannis et al., in Myocardial Ischemia: Mechanisms, Reperfusion Protection, M. Karmazyn, ed., Birkhuser Verlag (1996) at 236-284; H. S. Shamia et al., Med of Inflamm. 6. 175 (1987)). atherosclerosis (R. Ross, Nature, 362, 801 (1993)). inflammatory aortic aneurysms (N. Girardi et al., Ann. Thor Surg. 64. 251 (1997); D. I. Walker et al., Brit. J. Surg., 59, 609 (1972); R. L. Pennell et al., J. Vasc. Surg., 2, 859 (1985)), and restenosis following balloon angioplasty (see, R. Ross cited above). The cells involved with inflammation include leukocytes (i.e., the immune system cells - neutrophils, cosinophils, lymphocytes, monocytes, basophils, macrophages, dendritic cells, and mast cells), the vascular endothelium, vascular smooth muscle cells, fibroblasts, and myocytes.

The release of inflammatory cytokines such as tumor necrosis factor-alpha (TNFα) by leukocytes is a means by which the immune system combats pathogenic invasions, including infections. TNFα stimulates the expression and activation of adherence factors on leukocytes and endothelial cells, primes neutrophils for an enhanced inflammatory response to secondary stimuli and enhances adherent neutrophil oxidative activity. See, Sharma et al., cited above. In addition, macrophages/dendritic cells act as accessory cells processing antigen for presentation to lymphocytes. The lymphocytes, in turn, become stimulated to act as pro-inflammatory cytotoxic cells.

Generally, cytokines stimulate neutrophils to enhance oxidative (e.g., superoxide and secondary products) and nonoxidative (e.g., myeloperoxidase and other enzymes) inflammatory activity. Inappropriate and over-release of cytokines can produce counterproductive exaggerated pathogenic effects through the release of tissue-damaging oxidative and nonoxidative products (K. G. Tracey et al., J. Exp. Med., 167, 1211 (1988); and D. N. Männel et al., Rev. Infect. Dis., 9 (suppl. 5), S602-S606 (1987)). For example, TNFα can induce neutrophils to adhere to the blood vessel wall and then to migrate through the vessel to the site of injury and release their oxidative and non-oxidative inflammatory products.

Although monocytes collect slowly at inflammatory foci, given favorable conditions, the monocytes develop into long-term resident accessory cells and macrophages. Upon stimulation with an inflammation trigger, monocytes/macrophages also produce and secrete an array of cytokines (including TNFα), complement, lipids, reactive oxygen species, proteases and growth factors that remodel tissue and regulate surrounding tissue functions.

For example, inflammatory cytokines have been shown to be pathogenic in: arthritis (C. A. Dinarello, Semin. Immunol., 4, 133 (1992)); ischemia (A. Seekamp et al., Agents-Actions-Supp., 41, 137 (1993)); septic shock (D. N. Männel et al., Rev. Infect. Dis., 9 (suppl. 5), S602-S606 (1987)); asthma (N. M. Cembrzynska et al., Am. Rev. Respir. Dis., 147, 291 (1993)); organ transplant rejection (D. K. Imagawa et al., Transplantation, 51, 57 (1991); multiple sclerosis (H. P. Hartung, Ann. Neurol., 33, 591 (1993)); AIDS (T. Matsuyama et al., AIDS, 5, 1405 (1991)); and in alkali-burned eyes (F. Miyamoto et al., Opthalmic Res., 30, 168 (1997)). In addition, superoxide formation in leukocytes has been implicated in promoting replication of the human immunodeficiency virus (HIV) (S. Legrand-Poels et al., AIDS Res. Hum. Retroviruses, 6, 1389 (1990)).

It is well known that adenosine and some analogs of adenosine that nonselectively activate adenosine receptor subtypes decrease neutrophil production of inflammatory oxidative products (B. N. Cronstein et al., Ann. N.Y. Acad. Sci., 451, 291 (1985); P. A. Roberts et al., Biochem. J., 227, 669 (1985); D. J. Schrier et al., J. Immunol., 137, 3284 (1986); B. N. Cronstein et al., Clinical Immunol. and Immunopath., 42, 76 (1987); M. A. Iannone et al., in Topics and Perspective in Adenosine Research, E. Gerlach et al., eds., Springer-Verlag, Berlin, p. 286 (1987); S. T. McGarrity et al., J. Leukocyte Biol., 44, 411421 (1988); J. De La Harpe et al., J. Immunol., 143, 596 (1989); S. T. McGarrity et al., J. Immunol., 142, 1986 (1989); and C. P. Nielson et al., Br. J. Pharmacol., 97, 882 (1989)). For example, adenosine has been shown to inhibit superoxide release from neutrophils stimulated by chemoattractants such as the synthetic mimic of bacterial peptides, f-met-leu-phe (fMLP), and the complement component C₅a (B. N. Cronstein et al., J. Immunol., 135, 1366 (1985)). Adenosine can decrease the greatly enhanced oxidative burst of PMN (neutrophil) first primed with TNF-α and then stimulated by a second stimulus such as f-met-leu-phe (G. W. Sullivan et al., Clin. Res., 41, 172A (1993)). Additionally, it has been reported that adenosine can decrease the rate of HIV replication in a T-cell line (S. Sipka et al., Acta. Biochim. Biopys. Hung., 23, 75 (1988)). However, there is no evidence that *in vivo* adenosine has antiinflammatory activity (G. S. Firestein et al., Clin. Res., 41, 170A (1993); and B. N. Cronstein et al., Clin. Res., 41, 244A (1993)).

It has been suggested that there is more than one subtype of adenosine receptor on neutrophils that can have opposite effects on superoxide release (B. N. Cronstein et al., J. Clin. Invest., 85, 1150 (1990)). The existence of A_{2A} receptor on neutrophils was originally demonstrated by Van Calker et al. (D. Van Calker et al., Eur. J. Pharmacology, 206, 285 (1991)).

There has been progressive development of compounds that are more and more potent and/or selective as agonists of A_{2A} adenosine receptors (AR) based on radioligand binding assays and physiological responses. Initially, compounds with little or no selectivity for A_{2A} receptors were developed, such as adenosine itself or 5'-carboxamides of adenosine, such as 5'-N-ethylcarboxamidoadenosine (NECA) (B. N. Cronstein et al., J. Immunol., 135, 1366 (1985)). Later, it was shown that addition of 2-alkylamino substituents increased potency and selectivity, e.g., CV1808 and CGS21680 (M. F. Jarvis et al., J. Pharmacol. Exp. Ther., 251, 888 (1989)). 2-Alkoxy-substituted adenosine derivatives such as WRC-0090 are even more potent and selective as agonists at the coronary artery A_{2A} receptor (M. Ueeda et al., J. Med. Chem., 34, 1334 (1991)). The 2-alklylhydrazino adenosine derivatives, e.g., SHA 211 (also called WRC-0474) have also been evaluated as agonists at the coronary artery A_{2A} receptor (K. Niiya et al., J. Med. Chem., 35, 4557 (1992)).

There is one report of the combination of relatively nonspecific adenosine analogs, R-phenylisopropyladenosine (R-PIA) and 2-chloroadenosine (Cl-Ado) with a phosphodiesterase (PDE) inhibitor resulting in a lowering of neutrophil oxidative activity (M. A. Iannone et al., Topics and Perspectives in Adenosine Research, E. Garlach et al., eds., Springer-Verlag, Berlin, pp. 286-298 (1987)). However, R-PIA and Cl-Ado analogs are actually more potent activators of A₁ adenosine receptors than of A_{2A} adenosine receptors and, thus, are likely to cause side effects due to activation of A, receptors on cardiac muscle and other tissues causing effects such as "heart block."

R. A. Olsson et al. (U.S. Pat. No. 5,278,150) disclose selective adenosine A₂ receptor agonists of the formula: 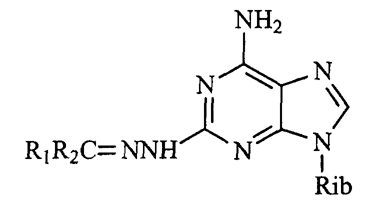 wherein Rib is ribosyl, R₁ can be H and R₂ can be cycloalkyl. The compounds are disclosed to be useful for treating hypertension, atherosclerosis and as vasodilators.

Olsson et al. (U.S. Pat. No. 5,140,015) disclose certain adenosine A₂ receptor agonists of formula: 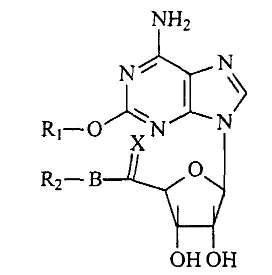 wherein C(X)BR₂ can be CH₂OH and R, can be alkyl- or alkoxyalkyl. The compounds are disclosed to be useful as vasodilators or an antihypertensives.

Linden et al. (U.S. Pat. No. 5,877,180) is based on the discovery that certain inflammatory diseases, such as arthritis and asthma, may be effectively treated by the administration of compounds which are selective agonists of A_{2A} adenosine receptors, preferably in combination with a Type IV phosphodiesterase inhibitor. An embodiment of the Linden et al. invention provides a method for treating inflammatory diseases by administering an effective amount of an A_{2A} adenosine receptor of the following formula: 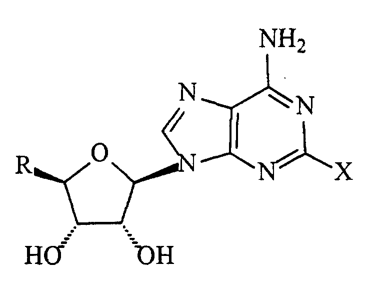 wherein R and X are as described in the patent.

In a preferred embodiment, the Linden et al. invention involves the administration of a Type IV phosphodiesterase (PDE) inhibitor in combination with the A_{2A} adenosine receptor agonist. The Type IV phosphodiesterase (PDE) inhibitor includes racemic and optically active 4-(polyalkoxyphenyl)-2-pyrrolidones of the following formula: 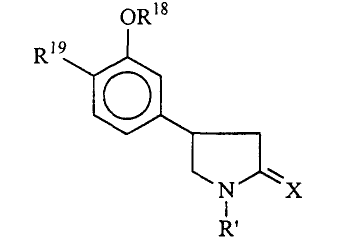 wherein R', R¹⁸, R¹⁹ and X are as disclosed and described in U.S. Pat. No. 4,193,926. Rolipram is an example of a suitable Type IV PDE inhibitor included within the above formula.

G. Cristalli (U.S. Pat. No. 5,593,975) discloses 2-arylethynyl, 2-cycloalkylethynyl or 2-hydroxyalkylethynyl derivatives, wherein the riboside residue is substituted by carboxy amino, or substituted carboxy amino (R₃HNC(O)-). 2-Alkynylpurine derivatives have been disclosed in Miyasaka et al. (U.S. Pat. No. 4,956,345), wherein the 2-alkynyl group is substituted with (C₃-C₁₆)alkyl. The'975 compounds are disclosed to be vasodilators and to inhibit platelet aggregation, and thus to be useful as anti-ischemic, anti-atherosclerosis and anti-hypertensive agents.

However, a continuing need exists for selective A₂ adenosine receptor agonists useful for therapeutic applications, that have reduced side effects.

### Summary of the Invention

The present invention comprises compounds which are useful for the treatment of inflammatory activity in mammalian tissue. The inflammatory tissue activity can be due to pathological agents or can be due to physical, chemical or thermal trauma, or the trauma of medical procedures, such as organ, tissue or cell transplantation, angioplasty (PCTA), inflammation following ischemia/reperfusion, or grafting. The present compounds comprise a novel class of 2-alkynyladenosine derivatives, substituted at the ethyne position by substituted cycloalkyl moieties. Preferably, the riboside residue is substituted at the 5'-position ("X") by an N-alkyl-(or cycloalkyl) carboxyamino ("aminocarbonyl") moiety. Thus, the present invention provides compounds which can be used for inhibiting the inflammatory response in a mammal, such as a human subject, and protecting the tissue subject to the response, by administering an effective amount of one or more compounds of the invention.

The compounds of the invention have the following general formula (I): 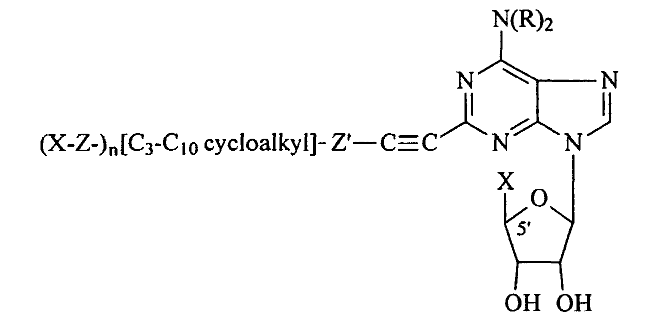 wherein
(a) each R is individually hydrogen, C₁-C₆ alkyl, C₃-C₇ cycloalkyl, phenyl or phenyl(C₁-C₃)-alkyl;
(b) X is -CH₂OH, -CO₂R², -OC(O)R², or C(O)NR³R⁴;
(c) each of R², R³ and R⁴ is individually H, C₁₋₆-alkyl; C₁₋₆-alkyl substituted with 1-3 C₁₋₆-alkoxy, C₃-C₇ cycloalkyl, C₁₋₆-alkylthio, halogen, hydroxy, amino, mono(C₁₋₆-alkyl)amino, di(C₁₋₆-alkyl)amino, or C₆₋₁₀-aryl, wherein aryl may be substituted with 1-3 halogen, C₁₋₆-alkyl, hydroxy, amino, mono(C₁₋₆-alkyl)amino, or di(C₁₋₆-alkyl)amino; C₆₋₁₀-aryl; or C₆₋₁₀-aryl substituted with 1-3 halogen, hydroxy, amino, mono(C₁₋₆-alkyl)amino, di(C₁₋₆-alkyl)amino or C₁₋₆-alkyl;
(d) Z and Z' are individually (C₁-C₆)alkyl, optionally interrupted by 1-3 S or nonperoxide O, or are absent, and n is 1-3; or a pharmaceutically acceptable salt thereof.

The invention provides a compound of formula I for use in medical therapy, preferably for use in treating or protecting tissue from inflammation such as an inflammatory response, as well as the use of a compound of formula I for the manufacture of a medicament for the treatment of an inflammatory response due to a pathological condition or symptom in a mammal, such as a human, which is associated with inflammation.

Although certain A_{2A} adenosine receptor agonists have been reported to be vasodilators, and thus to be useful to directly treat hypertension, thrombus, atherosclerosis and the like, the tissue-protective activity of the compounds of formula (I) is not suggested by the prior art.

The invention also includes the use of a combination of these compounds with type IV phosphodiesterase inhibitors for synergistic decreases in the inflammatory response of immune cells.

The invention also provides a pharmaceutical composition comprising an effective amount of the compound of formula 1, or a pharmaceutically acceptable salt thereof, in combination with a pharmaceutically acceptable diluent or carrier, and optionally, in combination with a Type IV phosphodiesterase (PDE) inhibitor. Preferably, the composition is presented as a unit dosage form.

Additionally, the invention provides a therapeutic method for preventing or treating a pathological condition or symptom in a mammal, such as a human, wherein the activity of A_{2A} adenosine receptors is implicated and agonism of said activity is desired, comprising administering to a mammal in need of such therapy, an effective amount of a compound of formula I, or a pharmaceutically acceptable salt thereof. It is believed that activation of A_{2A} adenosine receptors inhibits inflammation by effecting neutrophils, mast cells, monocytes/macrophages, T-cells and/or eosinophils. Inhibition of these inflammatory cells results in tissue protection following tissue insults.

Among the inflammatory responses that can be treated (including treated prophylactically) with a compound of formula I, optionally with a Type IV PDE inhibitor, are inflammation due to
(a) autoimmune stimulation (autoimmune diseases), such as lupus erythematosus, multiple sclerosis, infertility from endometriosis, type I diabetes mellitus including the destruction of pancreatic islets leading to diabetes and the inflammatory consequences of diabetes, including leg ulcers, Crohn's disease, ulcerative colitis, inflammatory bowel disease, osteoporosis and rheumatoid arthritis;
(b) allergic diseases such as asthma, hay fever, rhinitis, vernal conjunctivitis and other eosinophil-mediated conditions;
(c) skin diseases such as psoriasis, contact dermatitis, eczema, infectious skin ulcers, open wounds, cellulitis;
(d) infectious diseases including sepsis, septic shock, encephalitis, infectious arthritis, endotoxic shock, gram negative shock, Jarisch-Herxheimer reaction, shingles, toxic shock, cerebral malaria, bacterial meningitis, acute respiratory distress syndrome (ARDS), lyme disease, HIV infection, (TNFα-enhanced HIV replication, TNFα inhibition of reverse transcriptase inhibitor activity);
(e) wasting diseases: cachexia secondary to cancer and HIV;
(f) organ, tissue or cell transplantation (e.g., bone marrow, cornea, kidney, lung, liver, heart, skin, pancreatic islets) including transplant rejection, and graft versus host disease;
(g) adverse effects from drug therapy, including adverse effects from amphotericin B treatment, adverse effects from immunosuppressive therapy, e.g., interleukin-2 treatment, adverse effects from OKT3 treatment, adverse effects from GM-CSF treatment, adverse effects of cyclosporine treatment, and adverse effects of aminoglycoside treatment, stomatitis and mucositis due to immunosuppression;
(h) cardiovascular conditions including circulatory diseases induced or exasperated by an inflammatory response, such as ischemia, atherosclerosis, peripheral vascular disease, restenosis following angioplasty, inflammatory aortic aneurysm, vasculitis, stroke, spinal cord injury, congestive heart failure, hemorrhagic shock, ischemia/reperfusion injury, vasospasm following subarachnoid hemorrhage, vasospasm following cerebrovascular accident, pleuritis, pericarditis, and the cardiovascular complications of diabetes;
(i) dialysis, including pericarditis, due to peritoneal dialysis;
(j) gout; and
(k) chemical or thermal trauma due to burns, acid, alkali and the like.

Of particular interest and efficacy is the use of the present compounds to treat inflammatory responses due to organ, tissue or cell transplantation, i.e., the transplantation of allogeneic or xenogeneic tissue into a mammalian recipient, autoimmune diseases and inflammatory conditions due to circulatory pathologies and the treatment thereof, including angioplasty, stent placement, shunt placement or grafting. Unexpectedly, it was found that administration of one or more compounds of formula (I) was effective after the onset of the inflammatory response, e.g., after the subject was afflicted with the pathology or trauma that initiates the inflammatory response.

A method is also disclosed for measuring the response, or binding a compound of formula I at or to designated A_{2A} adenosine receptor sites comprising said receptors, *in vivo* or *in vitro*, with an amount of a compound of formula I effective to bind to said receptors. Tissue or cells comprising ligand bound receptor sites can be used to measure the selectively of test compounds for specific receptor subtypes, the amount of bioactive compound in blood or other physiological fluids, or can be used as a tool to identify potential therapeutic agents for the treatment of diseases or conditions associated with receptor site activation, by contacting said agents with said ligand-receptor complexes, and measuring the extent of displacement of the ligand and/or binding of the agent, or the cellular response to said agent (e.g., cAMP accumulation).

### Detailed Description of the Invention

The following definitions are used, unless otherwise described. Halo is fluoro, chloro, bromo, or iodo. Alkyl. alkoxy, aralkyl, alkylaryl, etc. denote both straight and branched alkyl groups; but reference to an individual radical such as "propyl" embraces only the straight chain radical, a branched chain isomer such as "isopropyl" being specifically referred to. Aryl includes a phenyl radical or an ortho-fused bicyclic carbocyclic radical having about nine to ten ring atoms in which at least one ring is aromatic. Heteroaryl encompasses a radical attached via a ring carbon of a monocyclic aromatic ring containing five or six ring atoms consisting of carbon and one to four heteroatoms each selected from the group consisting of non-peroxide oxygen, sulfur, and N(X) wherein X is absent or is H, O, (C₁-C₄)alkyl, phenyl or benzyl, as well as a radical of an ortho-fused bicyclic heterocycle of about eight to ten ring atoms derived therefrom, particularly a benz-derivative or one derived by fusing a propylene, trimethylene, or tetramethylene diradical thereto.

It will be appreciated by those skilled in the art that the compounds of formula (I) have more than one chiral center and may be isolated in optically active and racemic forms. Preferably, the riboside moiety of formula (I) is derived from D-ribose, i.e., the 3',4'-hydroxyl groups are alpha to the sugar ring and the 2' and 5' groups is beta (3R, 4S, 2R, 5S). When the two groups on the cyclohexyl group are in the 4-position, they are preferably *trans*. Some compounds may exhibit polymorphism. It is to be understood that the present invention encompasses any racemic, optically-active, polymorphic, or stereoisomeric form, or mixtures thereof, of a compound of the invention, which possess the useful properties described herein, it being well known in the art how to prepare optically active forms (for example, by resolution of the racemic form by recrystallization techniques, or enzymatic techniques, by synthesis from optically-active starting materials, by chiral synthesis, or by chromatographic separation using a chiral stationary phase) and how to determine adenosine agonist activity using the tests described herein, or using other similar tests which are well known in the art.

Specific and preferred values listed below for radicals, substituents, and ranges, are for illustration only; they do not exclude other defined values or other values within defined ranges for the radicals and substituents.

Specifically, (C₁-C₆)alkyl can be methyl, ethyl, propyl, isopropyl, butyl, iso-butyl, sec-butyl, pentyl, 3-pentyl, or hexyl. As used herein, the term "cycloalkyl" encompasses bycycloalkyl (norbornyl, 2.2.2-bicyclooctyl, etc.) and tricycloalkyl (adamantyl, etc.), optionally comprising 1-2 N, O or S. Cycloalkyl also encompasses (cycloalkyl)alkyl. Thus, (C₃-C₆)cycloalkyl can be cyclopropyl, cyclobutyl, cyclopentyl, or cyclohexyl; (C₃-C₆)cycloalkyl(C₁-C₆)alkyl can be cyclopropylmethyl, cyclobutylmethyl, cyclopentylmethyl, cyclohexylmethyl;, 2-cyclopropylethyl, 2-cyclobutylethyl, 2-cyclopentylethyl, or 2-cyclohexylethyl.

(C₁-C₆)alkoxy can be methoxy, ethoxy, propoxy, isopropoxy, butoxy, iso-butoxy, sec-butoxy, pentoxy, 3-pentoxy, or hexyloxy; (C₂-C₆)alkenyl can be vinyl, allyl, 1-propenyl, 2-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, or 5-hexenyl; (C₂-C₆)alkynyl can be ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, or 5-hexynyl; (C₁-C₆)alkanoyl can be acetyl, propanoyl or butanoyl; halo(C₁-C₆)alkyl can be iodomethyl, bromomethyl, chloromethyl, fluoromethyl, trifluoromethyl, 2-chloroethyl, 2-fluoroethyl, 2,2,2-trifluoroethyl, or pentafluoroethyl; hydroxy(C₁-C₆)alkyl can be hydroxymethyl, 1-hydroxyethyl, 2-hydroxyethyl, 1-hydroxypropyl, 2-hydroxypropyl, 3-hydroxypropyl, 1-hydroxybutyl, 4-hydroxybutyl, 1-hydroxypentyl, 5-hydroxypentyl, 1-hydroxyhexyl, or 6-hydroxyhexyl; (C₁-C₆)alkoxycarbonyl (CO₂R²) can be methoxycarbonyl, ethoxycarbonyl, propoxycarbonyl, isopropoxycarbonyl, butoxycarbonyl, pentoxycarbonyl, or hexyloxycarbonyl; (C₁-C₆)alkylthio can be methylthio, ethylthio, propylthio, isopropylthio, butylthio, isobutylthio, pentylthio, or hexylthio; (C₂-C₆)alkanoyloxy can be acetoxy, propanoyloxy, butanoyloxy, isobutanoyloxy, pentanoyloxy, or hexanoyloxy; aryl can be phenyl, indenyl, or naphthyl; and heteroaryl can be furyl, imidazolyl, triazolyl, triazinyl, oxazoyl, isoxazoyl, thiazolyl, isothiazoyl, pyraxolyl, pyrrolyl, pyrazinyl, tetrazolyl, puridyl (or its N-oxide), thientyl, pyrimidinyl (or its N-oxide), indolyl, isoquinolyl (or its N-oxide) or quinolyl (or its N-oxide).

A specific value for R is amino, monomethylamino or cyclopropylamino.

A specific value for R¹ is carboxy- or (C₁-C₄)alkoxycarbonyl-cyclohexyl(C₁-C₄)alkyl.

A specific value for R² is H or (C₁-C₄)alkyl, i.e., methyl or ethyl.

A specific value for R³ is H, methyl or phenyl.

A specific value for R⁴ is H, methyl or phenyl.

A specific value for Z is -CH₂- or -CH₂-CH₂-.

A specific value for X is CO₂R², (C₂-C₅)alkanoylmethyl or amido.

A specific value for n is 1.

Preferred compounds of formula (I) are those wherein each R is H, X is ethylaminocarbonyl and R' is 4-carboxycyclohexylmethyl (DWH-146a), R' is 4-methoxycarbonylcyclohexylmethyl (DWH-146e) or R' is 4-acetoxymethyl-cyclohexylmethyl (JMR-193). They are depicted below (DWH-146 (acid) and methylester (e)) and JMR-193.

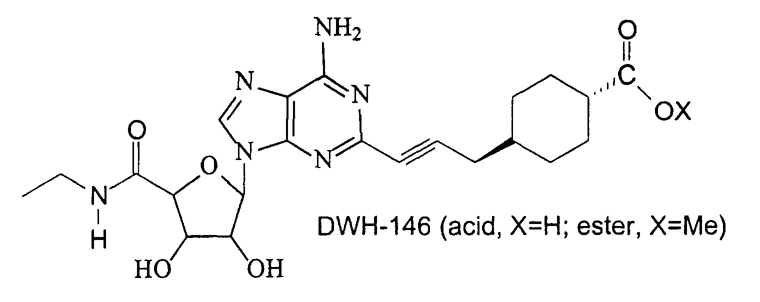

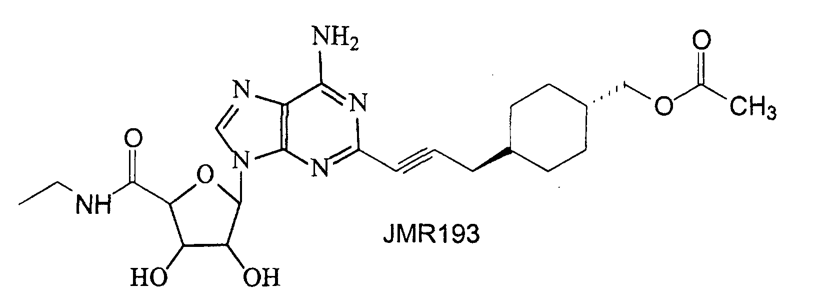

The synthesis of methyl 4[3-(6-amino-9(5-[(ethylamino)carbonyl]-3,4-dihydroxytetrahydro-Z-furanyl-9*H*-2-purinyl)-2-propynyl]-1-cyclohexanecarboxylate (DWH-146e) was accomplished by the cross coupling of an iodo-adenosine derivative (N-ethyl-1'-deoxy-1'-(amino-2-iodo-9*H*-purin-9-yl)-β-D-ribofuranuoramide) with methyl 4-(2-propynyl)-1-cyclohexanecarboxylate by utilization of a Pd¹¹ catalyst. The synthesis of the iodo-adenosine derivative was accomplished from guanosine. Guanosine is first treated with acetic anhydride, which acetalates the sugar hydroxyls, followed by the chlorination of position 6 with tetramethyl ammonium chloride and phosphorousoxychloride. Iodination of position 2 was accomplished via a modified Sandmeyer reaction, followed by displacement of the 6-Cl and sugar acetates with ammonia. The 2' and 3' hydroxyls were protected as the acetonide and the 5' hydroxyl was iodized to the acid with potassium permanganate. Deptrotection of the 2' and 3' acetonide, Fisher esterification of the 5' acid with ethanol and conversion of the resulting ethyl ester to the ethyl amide with ethylamine gave N-ethyl-1'-deoxy-1'-(amino-2-iodo-9*H*-purin-9-yl)-β-D-ribofuranuoramide.

The acetylene (methyl 4-(2-propynyl)-1-cyclohexanecarboxylate) was synthesized starting from *trans*-1,4-cyclohexanedimethanol. Initially the *trans*-diol was monotosylated followed by displacement of the tosylate with an acetylene anion. The hydroxyl of the resulting hydroxyl acetylene species was oxidized to the acid via Jones reagent followed by methylation with (trimethylsilyl)diazomethane to give methyl 4-(2-propynyl)-1-cyclohexanecarboxylate.

The cross-coupling reaction was performed under the following previously reported conditions. To a solution of *N,N*-dimethylformamide (0.5 mL), acetomtrile (1 mL), triethylamine (0.25 mL), and *N*-ethyl-1'-deoxy-1'-(amino-2-iodo-9*H*-purin-9-yl)-β-D-ribofuranuroamide (25 mg, 0.06 mmol) was added bis(triphenylphosphine)palladium dichloride (1 mg, 2 mol%) and copper(I)iodide (0-06 mg, .5 mol%). To the resulting mixture was added methyl 4-(2-propynyl)-1-cyclohexanecarboxylate (54 mg, 0.3 mmol) and the reaction was stirred under N₂ atmosphere for 16 hours. The solvent was removed under vacuum and the resulting residue was flash chromatographed in 20% methanol in chloroform (R_{*f*} = 0.45) to give 19 mg (off-white solid, mp 125°C (decomposed)) of methyl 4[3-(6-amino-9(5-[(ethylamino)carbonyl]-3,4-dihydroxytetrahydro-Z-furanyl)-9*H*-2-purinyl)-2-propynyl]-1-cyclohexanecarboxylate (DWH-146e).

DWH-146e and JMR193 are substantially more potent as inhibitors in inflammatory model systems than the reference compound, CGS21680 (2-[*p*-(carboxyethyl)-phenyl-ethylamino]5'-N-ethylcarboxamidoadenosine). For example, DWH-146e is about 80 times more potent at A_{2A} receptors and 40 times more selective for A_{2A} over A₃ receptors than is CGS21680.

Examples of pharmaceutically acceptable salts are organic acid addition salts formed with acids which form a physiological acceptable anion, for example, tosylate, methanesulfonate, malate, acetate, citrate, malonate, tartarate, succinate, benzoate, ascorbate, α-ketoglutarate, and α-glycerophosphate. Suitable inorganic salts may also be formed, including hydrochloride, sulfate, nitrate, bicarbonate, and carbonate salts.

Pharmaceutically acceptable salts may be obtained using standard procedures well known in the art, for example by reacting a sufficiently basic compound such as an amine with a suitable acid affording a physiologically acceptable anion. Alkali metal (for example, sodium, potassium or lithium) or alkaline earth metal (for example calcium) salts of carboxylic acids can also be made.

The compounds of formula I can be formulated as pharmaceutical compositions and administered to a mammalian host, such as a human patient in a variety of forms adapted to the chosen route of administration, i.e., orally or parenterally, by intravenous, intramuscular, topical or subcutaneous routes.

Thus, the present compounds may be systemically administered, e.g., orally, in combination with a pharmaceutically acceptable vehicle such as an inert diluent or an assimilable edible carrier. They may be enclosed in hard or soft shell gelatin capsules, may be compressed into tablets, or may be incorporated directly with the food of the patient's diet. For oral therapeutic administration, the active compound may be combined with one or more excipients and used in the form of ingestible tablets, buccal tablets, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 60% of the weight of a given unit dosage form. The amount of active compound in such therapeutically useful compositions is such that an effective dosage level will be obtained.

The tablets, troches, pills, capsules, and the like may also contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active compound may be incorporated into sustained-release preparations and devices.

The active compound may also be administered intravenously or intraperitoneally by infusion or injection. Solutions of the active compound or its salts can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations contain a preservative to prevent the growth of microorganisms.

The pharmaceutical dosage forms suitable for injection or infusion can include sterile aqueous solutions or dispersions or sterile powders comprising the active ingredient which are adapted for the extemporaneous preparation of sterile injectable or infusible solutions or dispersions, optionally encapsulated in liposomes. In all cases, the ultimate dosage form must be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filter sterilization. In the case of sterile powders for the preparation of sterile injectable solutions, the preferred methods of preparation are vacuum drying and the freeze drying techniques, which yield a powder of the active ingredient plus any additional desired ingredient present in the previously sterile-filtered solutions.

For topical administration, the present compounds may be applied in pure form, i.e., when they are liquids. However, it will generally be desirable to administer them to the skin as compositions or formulations, in combination with a dermatologically acceptable carrier, which may be a solid or a liquid.

Useful solid carriers include finely divided solids such as talc, clay, microcrystalline cellulose, silica, alumina and the like. Useful liquid carriers include water, alcohols or glycols or water-alcohol/glycol blends, in which the present compounds can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers.

Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Examples of useful dermatological compositions which can be used to deliver the compounds of formula I to the skin are disclosed in Jacquet et al. (U.S. Pat. No. 4,608,392), Geria (U.S. Pat. No. 4,992,478), Smith et al. (U.S. Pat. No. 4,559,157) and Wortzman (U.S. Pat. No. 4,820,508).

Useful dosages of the compounds of formula I can be determined by comparing their *in vitro* activity, and *in vivo* activity in animal models. Methods for the extrapolation of effective dosages in mice, and other animals, to humans are known to the art; for example, see U.S. Pat. No. 4,938,949. Useful dosages of Type IV PDE inhibitors are known to the art. For example, see, U.S. Pat. No. 5,877,180, Col. 12.

Generally, the concentration of the compound(s) of formula (I) in a liquid composition, such as a lotion, will be from about 0.1-25% wt-%, preferably from about 0.5-10 wt-%. The concentration in a semi-solid or solid composition such as a gel or a powder will be about 0.1-5 wt-%, preferably about 0.5-2.5 wt-%.

The amount of the compound, or an active salt or derivative thereof, required for use in treatment will vary not only with the particular salt selected but also with the route of administration, the nature of the condition being treated and the age and condition of the patient and will be ultimately at the discretion of the attendant physician or clinician.

In general, however, a suitable dose will be in the range of from about 0.5 to about 100 µg/kg, e.g., from about 10 to about 75 µg/kg of body weight per day, such as 3 to about 50 µg per kilogram body weight of the recipient per day, preferably in the range of 6 to 90 µg/kg/day, most preferably in the range of 15 to 60 µg/kg/day.

The compound is conveniently administered in unit dosage form; for example, containing 5 to 1000 µg, conveniently 10 to 750 µg, most conveniently, 50 to 500 µg of active ingredient per unit dosage form.

Ideally, the active ingredient should be administered to achieve peak plasma concentrations of the active compound of from about 0.1 to about 10 nM, preferably, about 0.2 to 10 nM, most preferably, about 0.5 to about 5 nM. This may be achieved, for example, by the intravenous injection of a 0.05 to 5% solution of the active ingredient, optionally in saline, or orally administered as a bolus containing about 1-100 µg of the active ingredient. Desirable blood levels may be maintained by continuous infusion to provide about 0.01-5.0 µg/kg/hr or by intermittent infusions containing about 0.4-15 µg/kg of the active ingredient(s).

The desired dose may conveniently be presented in a single dose or as divided doses administered at appropriate intervals, for example, as two, three, four or more sub-doses per day. The sub-dose itself may be further divided, e.g., into a number of discrete loosely spaced administrations; such as multiple inhalations from an insufflator or by application of a plurality of drops into the eye. For example, it is desirable to administer the present compositions intravenously over an extended period of time following the insult that gives rise to inflammation.

The ability of a given compound of the invention to act as an A_{2A} adenosine receptor agonist (or antagonist) may be determined using pharmacological models which are well known to the art, or using tests described below.

The invention will be further described by reference to the following detailed examples.

The following are trade marks : Celite, Ficoll, Zeneca and ICN.

**Example 1. *Trans*-(1-[4-hydroxymethyl)cyclohexyl]methyl)-4-methylbenzenesulfonate (5.2).** Sodium hydride (1.68 g, 70 mmol) was added to a solution of 10 g (70 mmol) [4-(hydroxymethyl)cyclohexyl]methan-1-ol (**5.1**) in 700 mL of tetrahydrofuran and stirred for 1 hour p-toluenesulfonyl chloride (13.3 g, 70 mmol) was then added and the reaction mixture was refluxed for 5 hours. The reaction was then cooled to 0°C and slowly quenched with water until there is no more reactive hydride. Once the hydride was quenched, the reaction mixture was diluted with ether (700 mL) and extracted 2 times with 10% aqueous potassium carbonate (700 mL). The organics were dried using sodium sulfate and the solvent was removed under reduced pressure. The product was purified by chromatography on silica gel column eluting with acetone-dichloromethane (5:95) to give **5.2** (35%). ¹H NMR (300 MHz, CDCl₃) δ 7.75 (d, *J*= 8.3 Hz, 2H), 7.32 (d, *J*= 8.1 Hz, 2H), 3.79 (d, *J*= 6.35 Hz, 2H), 3.39 (d, J= 6.35 Hz, 2H), 2.42 (s, 3H), 1.75 (m, 4H), 1.59 (m, 1H), 1.37 (m, 1H), 0.9 (m, 4H). ¹³C NMR (300 MHz, CDCl₃) δ 145.3, 133.4, 130.3, 130.3, 128.3, 128.3, 75.8, 68.5, 40.6, 37.8, 28.9, 28.9, 28.9, 28.9, 22.1.

**Example 2. (4-prop-2-ynyleyclohexyl)methan-1-ol (5.3).** Lithium acetylide ethylenediamine complex (90%) (6.4 g, 70 mmol) was added very slowly to a solution of **5.2** (3 g, 10 mmol) in 40 mL of dimethylsulfoxide. The reaction mixture was allowed to stir for 5 days and then slowly quenched at 0°C with water. This mixture was diluted with ether (300 mL) and extracted 3 times with saturated aqueous ammonium chloride (200 mL). The organics were dried with sodium sulfate. The solvent was removed under reduced pressure and the product was purified by chromatography on silica gel column eluting with ethyl acetate-hexanes (20:80) to give **5.3** (85%). ¹H NMR (300 MHz, CDCl₃) δ 3.41 (d, *J* = 6.5 Hz, 2H), 2.07 (dd, *J*= 2.5, 6.5 Hz, 2H), 1.96-1.75 (m, 5H), 1.41 (m, 2H), .095 (m, 4). ¹³C NMR (300 MHz, CDCl₃) δ 83.8, 69.6, 68.9, 40.7, 37.7, 32.3, 32.3, 29.6, 29.6, 26.5.

**Example 3. 4-prop-2-ynylcyclohexanecarboxylic acid (5.4).** A solution of chromium trioxide (1.1 g, 11 mmol) in 1.5 M sulfuric acid (40 mL, 27 mmol) was maintained at 0°C while **5.3** (0.46 g, 3 mmol) in 80 mL of acetone was added over 2 hours. The reaction was then stirred for an additional 2 hours at room temperature. The reaction mixture was diluted with ether (200 mL) and extracted 2 times with water. The organics were dried with sodium sulfate. The solvent was removed under reduced pressure and the product was purified by chromatography on silica gel column eluting with acetone-dichloromethane (70:30) to give **5.4** (75%). ¹H NMR (300 MHz, CDCl₃) δ 2.24 (dt, *J=* 3.66, 12.1 Hz, 1H), 2.10 (dd, *J=* 2.7, 6.5 Hz, 2H), 2.04-1.89 (m, 5H), 1.76 (d, *J* = 2.3 Hz, 1H), 1.43 (dq, *J =* 3.28, 13.1 Hz, 2H), 1.03 (dq, *J =* 3.28, 13.1 Hz, 2H). ¹³C NMR (300 MHz, CDCl₃) δ 183.2, 83.3, 69.9, 43.4, 36.7, 31.8, 28.9, 26.3.

**Example 4. Methyl 4-prop-2-ynylcyclohexanecarboxylate (5.5).** (Trimethylsilyl)diazomethane (2.0 M) solution in hexanes (1 mL, 2 mmol) was added to a solution of **5.4** (0.34 g, 2 mmol) in 15 mL of methanol:dichloromethane (3:7). The solvents were removed under reduced pressure resulting in 100% conversion of starting material to product. ¹H NMR (300 MHz, CDCl₃) δ 2.24 (dt, *J=* 3.66, 12.1 Hz, 1H), 2.10 (dd, *J=* 2.7, 6.5 Hz, 2H), 2.06 (dd, *J* = 1.54, 6.54 Hz, 1H), 2.00-1.89 (m, 3H), 1.76 (d, *J=* 2.3 Hz, 1H), 1.43 (dq, *J =* 3.28, 13.1 Hz, 2H), 1.03 (dq, *J=* 3.28, 13.1 Hz, 2H). ¹³C NMR (300 MHz, CDCl₃) δ 176.8, 83.3, 69.8, 51.9, 43.4, 36.7, 31.9, 29.2, 26.3.

**Example 5. [(2R,3R,4R,5R)-3,4-diacetyloxy-5-(2-amino-6-oxohyropurin-9-yl)oxolan-2-yl]methyl acetate (6.2).** A suspension of 113 g (0.4 mol) of dry guanosine (**6.1**), acetic anhydride (240 mL, 2.5 mol), dry pyridine (120 mL) and dry DMF (320 mL) was heated for 3.75 hours at 75°C without allowing the temperature to exceed 80°C. The clear solution was then transferred to a 3L Erlenmyer flask and filled with 2-propanol. Upon cooling the solution to room temperature crystallization was initiated and allowed to proceed at 4°C overnight. The white solid filtrate was filtered, washed with 2-propanol and recrystallized from 2-propanol to give **6.2** (96%). ¹H NMR (300 Mhz, CDCl₃) δ 8.20 (s, 1H, H-8), 6.17 (d, *J* = 5.41 Hz, 1 H, H-1') 5.75 (t, *J=* 5.39 Hz, 1H, H-2'), 5.56 (t, *J =* 5.0, H-3'), 4.41 (m, 3H, H-4',5'), 2.14 (s, 3H, Ac), 2.11 (s, 3H, Ac), 2.10 (s, 3H, Ac). ¹³C NMR (300 MHz, CD₃OD) δ 171.0, 170.3, 1702, 157.7, 154.8,152.4, 136.7, 117.7, 85.5, 80.4, 73.0, 71.3, 64.0, 31.3, 21.2, 21.0.

**Example 6. [(2R,3R,4R,5R)-3,4-diacetyloxy-5-(2-amino-6-chloropnrin-9-yl)oxolan-2-yl]methyl acetate (6.3).** To a 1000 mL flask was added 80 g (0.195 mol) [(2R,3R,4R,5R)-3-4-diacetyloxy-5-(2-amino-6-oxohyropurin-9-yl)oxolan-2-yl]methyl acetate (**6.2**), tetramethylammonium chloride (44 g, 0.4 mol), anhydrous acetonitrile (400 mL) and N,N-dimethlaniline (25 mL). The flask was placed in an ice salt bath and cooled to 2°C. To this solution was added dropwise POCl₃ (107 mL 1.15 mol) at a rate that maintained the temperature below 5°C (45 minutes). The flask was then removed from the ice bath, outfitted with a condenser, placed in an oil bath and allowed to reflux for 10 minutes whereas the solution changed to a red/brown color. The solvent was then removed under reduced pressure to yield an oily residue which was transferred to a beaker containing 1000 g of ice and 400 mL of CHCl₃ and allowed to stir for 1.5 hours to decompose any remaining POCl₃. The organic phase was then removed and the aqueous phase extracted with 3 × 50 mL of CHCl₃ and pooled with the organic phase. The pooled organic was then back extracted with 50 mL of water followed by stirring with 200 mL of saturated NaHCO₃. The organic was further extracted with NaHCO₃ until the aqueous extract was neutral (2X). The organic was finally extracted with brine and then dried over MgSO₄ for 16 hours. To the solution was added 800 mL of 2-propanol after which the solution was concentrated under reduced pressure. To the oily solid was added 200 mL of 2-propanol and the solution was refrigerated overnight. The crystalline product was filtered, washed, and allowed to dry overnight to give **6.3** (77%). ¹H NMR (300 MHz, CD₃OD) δ 8.31 (s, 1H, H-8), 7.00 (s, 2H, NH₂) 6.06 (d, *J*= 5.8 Hz, 1H, H-1'), 5.83 (t, *J*= 6.16 Hz, 1H, H-2'), 5.67 (m, 1H, H-3'), 4.29 (m, 3H, H-4',5'), 2.07 (s, 3H, Ac), 1.99 (s, 3H, Ac), 1.98 (s, 3H, Ac). ¹³C NMR (300 MHz, CD₃OD) δ 171.0, 170.4, 170.2, 160.8, 154.6, 150.8, 142.2, 124.5, 85.8, 80.6, 72.8, 71.2, 63.9, 21.4, 21.3, 21.1.

**Example 7. [(2R,3R,4R,5R)-3,4-diacetyloxy-5-(6-chloro-2-iodopurin-9-yl)oxolan-2-yl]methyl acetate (6.4).** Isoamyl nitrite (5 mL, 37 mmol) was added to a mixture of 5.12 g (12 mmol) [(2R,3R,4R,5R)-3-,4-diacetyloxy-5-(2-amino-6-chloropurin-9-yl)oxolan-2-yl]methyl acetate (**6.3**), I₂ (3.04 g, 12 mmol), CH₂I₂ (10 mL, 124 mmol), and CuI (2.4 g, 12.6 mmol) in THF (60 mL). The mixture was heated under reflux for 45 minutes and then allowed to cool to room temperature. To this solution was added 100 ml of sat. Na₂S₂O₃ which removed the reddish color due to iodine. The aqueous was extracted 3X with chloroform, which was pooled, dried over MgSO₄, and concentrated under reduced pressure. The product was then purified over a silica gel column using CHCl₃-MeOH (98:2) to collect [(2R,3R,4R,5R)-3,4-diacetyloxy-5-(6-chloro-2-iodopurin-9-yl)oxolan-2-yl]methyl acetate (**6.4**) (80% crystallized from EtOH). ¹H NMR (300 MHz, CDCl₃) δ 8.20 (s, 1H H-8), 6.17 (d, *J* = 5.41 Hz, 1H, H-1'), 5.75 (t, *J* = 5.39 Hz, 1H, H-2'), 5.56 (t, *J* = 5.40 Hz, 1H, H-3'), 4.38 (m, 3H, H-4',5'), 2.14 (s, 1H, Ac), 2.11 (s, 1H, Ac), 2.10 (s, 1H, Ac).

**Example 8. (4S,2R,3R,5R)-2-(6-amino-2-iodopurin-9-yl)-5-(hydroxymethyl)oxolane-3,4-diol (6.5).** To a flask containing 6.0 g (11.1 mmol) [(2R,3R,4R,5R)-3,4-diacetyloxy-5-(6-chloro-2-iodopurin-9-yl)oxolan-2-yl]methyl acetate (**6.4**) was added 100 ml of liquid NH₃ at -78°C and the solution was allowed to stir for 6 hours. After which time it was allowed to come to r.t. overnight with concurrent evaporation of the NH₃ to yield a brown oil. The product was crystallized from hot isopropanol to give **6.5** (80%), m.p. 143-145°C, r.f. = 0.6 in 20% MeOH/CHCl₃. ¹H NMR (300 MHz, DMSO-d₆) δ 8.24 (s, 1H), 7.68 (s, 2H), 5.75 (d, *J* = 6.16, 1H), **5.42** (d, *J =* 5.40 Hz, 1H), 5.16 (d, *J* = 4.62 Hz, 1H), 4.99 (t, *J =* 5.39 Hz, 1H), 4.67 (d, *J =* 4.81 Hz, 1H), 4.06 (d, *J* = 3.37 Hz, 1H), 3.89 (m, 1H), 3.54 (m, 2H).

**Example 9. [(1R,2R,4R,5R)-4-(6-amino-2-iodopurin-9-yl)-7-7-dimethyl-3,6,8-trioxabicyclo[3.3.0]oct-2-yl]methan-1-ol (6.6).** To a solution of 2.0 g (5.08 mmol) (4S,2R,3R,5R)-2-(6-amino-2-iodopurin-9-yl)-5(hydroxymethyl)oxolane-3,4-diol **(6.6)** in 100 mL acetone was added 9.6 g of p-toluenesulfonic acid and 5 ml of dimethoxypropane. The reaction was stirred at room temperature for 1 hour at which time 15 g of NaHCO₃ and then stirred for an additional 3 hours. the residue was filtered and washed 2X with EtOAc. The filtrate was then concentrated under reduced pressure. The residue was chromatographed on a silica gel column with MeOH-CHCl₃ (1:99) to give **6.6** (72%) as a solid, m.p. 185-187°C. ¹H NMR (300 MHz, DMSO-d₆) δ 8.22 (s, 1H, H-8), 7.69 (s, 2H), NH₂), 6.00 (d, *J =* 2.70 Hz, 1H, H-1'), 5.21 (m, 1H, H-2'), 5.07 (bs, 1H, OH), 4.88 (m, 1H, H-3'), 4.13 (m, 1H, H-4'), 3.47 (m, 2H, H-5'), 1.49 and 1.28 (s, 3H, C(CH₃)₂).

**Example 10. (2S,1R,4R,5R)-4-(6-amino-2-iodopurin-9-yl)-7,7-dimethyl-3,6,8-trioxabicyclo[3.3.0]octane-2-carboxylic acid (6.7).** To a stirred solution of 1.6 g (3.7 mmol) of [(1R,2R,4R,5R)-4-(6-amino-2-iodopurin-9-yl)-7-7-dimethyl-3,6,8-trioxabicyclo[3.3.0]oct-2-yl]methan-1-ol (6.6) in 200 mL of H₂O was added 0.60 g of KOH and, dropwise, a solution of 1.70 g (10.8 mml) of KMnO₄ in 50 mL of H₂O. The mixture was set aside in the dark at room temperature for 225 hours. The reaction mixture was then cooled to 5-10°C and decolorized by a solution of 4 mL of 30% H₂O2 in 16 mL of water, while the temperature was maintained under 10°C using an ice-salt bath. The mixture was filtered through Celite and the filtrate was concentrated under reduced pressure to about 10 mL and then acidified to pH 4 with 2N HCl. The resulting precipitate was filtered off and washed with ether to yield **6.7** (70%) after drying as a white solid, m.p. 187-190°C. ¹H NMR (300 MHz, DMSO-d₆) δ 8.11 (s, 1H, H-8), 7.62 (s, 2H, NH₂), 7.46 (s, 1H, COOH), 6.22 (s, 1H, H-1'), 5.42 (d, *J* = 5.71 Hz, 1H, H-2'), 5.34 (d, *J* = 6.16 Hz, 1H, H-3'), 4.63 (s, 1H, H-4'), 1.46 and 1.30 (s, 3H, C(CH₃)₂).

**Example 11. (2S,3S,4R,5R)-5-(6-amino-2-iodopurin-9-yl)-3,4**-dihydroxyoxolane-2-carboxylic acid (6.8). A solution of 1.72 g (3.85 mmol) of (2S,1R,4R,5R)-4-(6-amino-2-iodopurin-9-yl)-7,7-dimethyl-3,6,8-trioxabicyclo[3.3.0]octane-2-carboxylic acid (**6.7**) in 80 mL of 50% HCOOH was stirred at 80°C for 1.5 hours. The reaction mixture was evaporated under reduced pressure, dissolved in H₂O, and the solution was evaporated again. This process was repeated until there was no odor of formic acid in the residue. Recrystallization from water yielded 1.33 g (85%) **6.8** as a white solid, m.p. 221-223°C, dec. ¹H NMR (300 MHz, DMSO-d₆) δ 8.31 (s, 1H, H-8), 7.68 (s, 2H, NH₂), 5.90 (d, *J* = 6.55 Hz, 1H, H-1'), 4.42 (m, 1H, H-2'), 4.35 ( d, *J* = 2.31 Hz, 1H, H-4'), 4.22 (m, 1H, H-3').

**Example 12. [(2S,3S,4R,5R)-5-(6-amino-2-iodopurin-9-yl)-3,4-dihydroxyoxolan-2-yl]-N-ethyicarboxamide (6.9).** To a cooled (5°C) and stirred solution of 1.29 g (3.17 mmol) of (2S,3S,4R,5R)-5-(6-amino-2-iodopurin-9-yl)-3,4-dihydroxyoxolane-2-carboxylic acid **(6.8)** in 150 mL of absolute ethanol was added dropwise 1.15 mL of ice-cooled SOCl₂. The mixture was stirred at room temperature overnight and then brought to pH 8 with saturated aqueous NaHCO₃. The mixture was filtered, and then the filtrate was concentrated under reduced pressure to yield a white solid which was dried and then redissolved in 20 mL of dry ethylamine at -20°C for 3 hours and then at room temperature overnight. The reaction mixture was diluted with absolute ethanol, and the precipitated product was filtered off and washed with dry ether to give 530 mg (72%) of **6.9** as a pure solid, m.p. 232-234°C. ¹H NMR (300 MHz, DMSO-d₆) δ 8.34 (s, 1H, H-8), 8.12 (t, 1H, NH), 7.73 (s, 2H, NH₂), 5.85, (d, *J*= 6.93 Hz, 1H, H-1'), 4.54 (m, 1H, H-2'), 4.25 (d, *J =* 1.92 Hz, 1H, H-4'), 4.13 (m, 1H, H-3'), 3.28 (m, 2H, CH₂CH₃), 1.00 (t, *J*= 7.2 Hz, 3H, CH₂CH₃).

**Example 13. Methyl-4-(3-{9-[(4S,5S,2R,3R)-5-(N-ethylcarbamoyl)-3,4-dihydroxyoxolan-2-yl]-6-aminopurin-2-yl)}prop-2-ynyl)cyclohexanecarboxylate (DWH-146e).** To a degassed solution of 25 mg (0.063 mmol) of [(2S,3S,4R,5R)-5-(6-amino-2-iodopurin-9-yl)-3,4-dihydroxyoxolan-2-yl]-N-ethylcarboxamide (**6.9**), 16.9 mg (0.094 mmol) (**5.5**), and 0.75 mg CuI in 5 mL each of triethyl amine (TEA) and acetonitrile was added 15 mg of Pd(PPh₃)₄. The solution was stirred for 24 hours at 70°C after which time the solution was filtered through celite and chromatographed on silica gel with MeOH-CHCl₃ (5:95) to give **DWH-146e** (24%).

**Example 14. (4-prop-2-ynylcyclohexyl)methyl acetate (5.6).** Acetic anhydride (0.92 mL, 8.25 mmol) and pyridine (.2 mL, 2.5 mmol) were added to a solution of 5.3 (250 mg, 1.65 mmol) in 25 mL ether. The reaction was allowed to stir at ambient temperature for 24 hours. Water was added to the reaction and the organic was further extracted with 10% NaHCO₃. The organic layer was dried with MgSO₄ and evaporated. The residue was chromatographed on silica gel with EtOAc-Hexanes (5:95) to yield 5.6 (47%).

**Example 15. [4-(3-{9-(4S,5S,2R,3R)-5-(N-ethylcarbamoyl)-3,4-dihydroxyoxolan-2-yl]-6-aminopurin-2-yl}prop-2-ynyl)cyclohexyl]methyl acetate (JMR193).** To a degassed solution of 125 mg (0.29 mmol) of [(2S,3S,4R,5R)-5-(6-amino-2-iodopurin-9-yl)-3,4-dihydroxyoxolan-2-yl]-N-ethylcarboxamide (6.9), 150 mg (0.77 mmol) (5.6), and 1.0 mg CuI in 1.3 mL of TEA and 4 mL DMF was added to 25 mg of Pd(PPh₃)₄. The solution was stirred for 72 hours at 60°C after which time the solution was filtered through celite and chromatographed on silica gel with MeOH-CHCl₃ (5:95) to give JMR193 (10%).

### Example 16. [(2S,3S,4R,5R)-5-(6-amino-2-{3-[4-(hydroxymethyl)-cyclohexyl]prop-2-ynyl}purin-9-yl)-3,4-dihydroxyoxolan-2-yl]-N-ethylcarboxamide.

**A. (4-prop-2-ynylcyclohexyl)methan-1-ol.** A lithium acetylide ethylenediamine complex (90%) (6.4 g, 70 mmol) was added very slowly to a solution of *trans*-[4-(hydroxymethyl)cyclohexyl]methyl 4-methylbenzenesulfonate (3 g, 10 mmol) in 40 mL of dimethylsulfoxide. The reaction mixture was allowed to stir for 5 days and then slowly quenched at 0° C with water. This mixture was diluted with ether (300 mL) and washed 3 times with saturated aqueous ammonium chloride (200 mL). The organics were dried with sodium sulfate. The solvent was removed under reduced pressure. The product was purified by chromatography on silica gel column eluting with ethyl acetate-hexanes (20:80) to furnish the product (85%). ¹H NMR (300 MHz, CDCl₃) d 3.41 (d, *J =* 6.5 Hz, 2H), 2.07 (dd, *J*= 2.5, 6.5 Hz, 2H), 1.96-1.75 (m, 5H), 1.41 (m, 2H), .095 (m, 4). ¹³C NMR (300 MHz, CDCl₃) d 83.8, 69.6, 68.9, 40.7, 37.7, 32.3, 32.3, 29.6, 29.6, 26.5.

**B. [(2S,3S,4R,5R)-5-(6-amino-2-{3-[4-(hydroxymethyl)cyclohexyl]-prop-1-ynyl}purin-9-yl)-3,4-dihydroxyoxolan-2-yl]-N-ethylcarboxamide (JMR2037).** Pd(PPh₃)₄, 10 mg, was added to a degassed solution of 28mg (0.065mmol) of [(2S,3S,4R,5R)-5-(6-amino-2-iodopurin-9-yl)-3,4-dihydroxyoxolan-2-yl]-N-ethylcarboxamide, 30 mg (0.20 mmol) of (4-prop-2-ynylcyclohexyl)methan-1-ol, and 1.0 mg CuI in 1 mL of triethyl amine (TEA), 1 mL DMF, and 1 mL acetonitrile. The solution was stirred for 60 hours at room temperature after which time the solution was filtered through celite and chromatographed on silica gel with MeOH-CHCl₃ (7:93) to give 5mg (17%) JMR2037. The title compound was tested using the binding assays disclosed herein and found to bind to recombinant human A_{2A} receptors, Ki of 694 ± 69 nM.

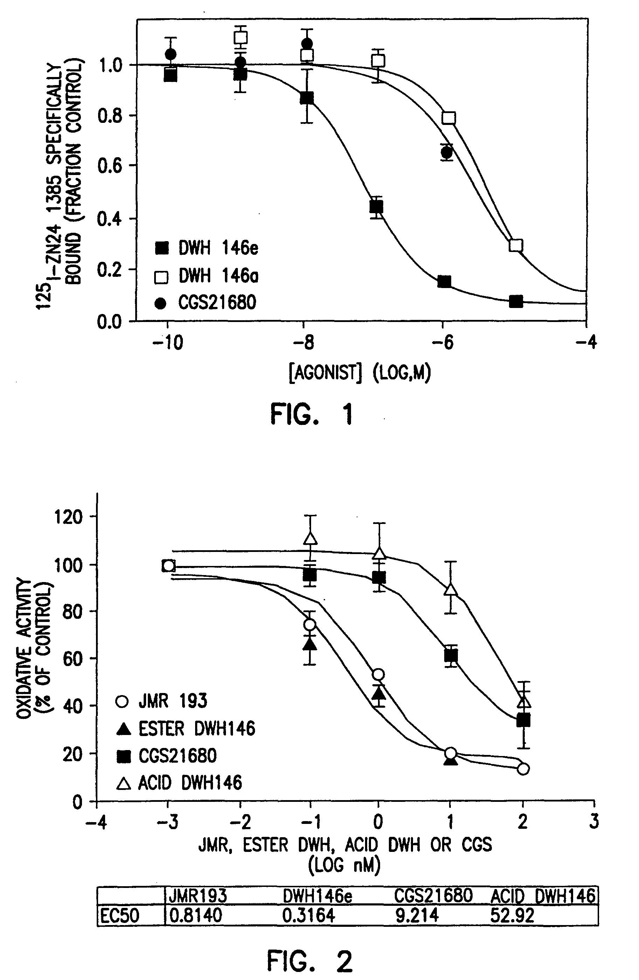

**Example 17. Radioligand Binding Studies.** Binding to A_{2A} receptors was evaluated with the radioligand ¹²⁵I-ZM241385. Figure 2B depicts the competition by selective agonists for binding to recombinant human A_{2A} adenosine receptors. DWH-146e is highly selective for the recombinant human A_{2A} (hA2A) subtype. Selectivity for the A₃ receptor (not shown) is less impressive, but still about 50-fold. DWH-146e is about 5 and 50 times more potent than WRC0470 and CGS21680, respectively (Fig. 1). An unexpected and interesting finding is that the ester, DWH-146e also is about 50 times more potent than the acid, DWH-146a (Fig. 1).

### Example 17A. Effect of DWH-146e and JMR193 on Neutrophil Oxidative Activity

### A. Materials.

f-met-leu-phe (fMLP), luminol, superoxide dismutase, cytochrome C, fibrinogen, adenosine deaminase, and trypan blue were obtained from Sigma Chemical. Ficoll-hypaque was purchased from ICN (Aurora, OH), and Cardinal Scientific (Santa Fe, NM) and Accurate Chemicals and Scientific (Westerbury, NY). Endotoxin (lipopolysaccharide; *E. coli* K235) was from List Biologicals (Campbell, CA). Hanks balanced salt solution (HBSS), and limulus amebocyte lysate assay kit were from BioWittaker (Walkersville, MD). Human serum albumin (HSA) was from Cutter Biological (Elkhart, IN). Recombinant human tumor necrosis factor-alpha was supplied by Dianippon Pharmaceutical Co. Ltd. (Osaka, Japan). ZM241385 (4-(2-[7-amino-2-(2-furyl)-[1,2,4]-triazolo[2,3-a][1,3,5]triazin-5-yl amino]ethyl)phenol) was a gift from Simon Poucher, Zeneca Pharmaceuticals, Cheshire, UK. Stock solutions (1 mM and 10 mM in DMSO) were made and stored at -20°C.

### B. Human neutrophil preparation

Purified neutrophils (∼98% neutrophils and >95% viable by trypan blue exclusion) containing <1 platelet per 5 neutrophils and < 50 pg/ml endotoxin (limulus amebocyte lysate assay) were obtained from normal heparinized (10 U/ml) venous blood by a one step Ficoll-hypaque separation procedure (A. Ferrante et al., J. Immunol. Meth., 36, 109 (1980)).

### C. Release of inflammatory reactive oxygen species from primed and stimulated human neutrophils Chemiluminescence

Luminol-enhanced chemiluminescence, a measure of neutrophil oxidative activity, is dependent upon both superoxide production and mobilization of the lysosomal granule enzyme myeloperoxidase. The light is emitted from unstable high-energy oxygen species generated by activated neutrophils. Purified neutrophils (5-10 × 10⁵/ml) were incubated in Hanks balanced salt solution containing 0.1% human serum albumin (1 ml) with or without DWH-146a, DWH-146e, CGS21680, or JMR193 with or without rolipram and with or without tumor necrosis factor-alpha (1 U/ml) for 30 minutes at 37°C in a shaking water bath. Then luminol (1 × 10⁻⁴ M) enhanced f-met-leu-phe (1 mcM) stimulated chemiluminescence was read with a Chronolog® Photometer (Crono-log Corp., Havertown, PA) at 37°C for 2-4 minutes. Chemiluminescence is reported as relative peak light emitted (= height of the curve) compared to samples with tumor necrosis factor-alpha and without DWH, JMR or rolipram.

### D. Results

As shown in Figure 2, JMR193 and DWH-146e both decreased tumor necrosis factor-alpha-primed f-met-leu-phe-stimulated human neutrophil oxidative activity as measured by luminol-enhanced chemiluminescence more effectively than the adenosine A_{2A} receptor agonist CGS21680. The horizonal axis gives the concentration of CGS21680, DWH-146a, DWH-146e or JMR193 (log nM). The vertical axis gives the resulting peak human neutrophil activity as relative amount of stimulated release of reactive oxygen species as measured with luminol-enhanced chemiluminescence compared to control samples which were not primed with tumor necrosis factor-alpha. Means SEM (n=4-5 separate experiments).

The data below the horizontal axis in Fig. 2 gives the EC₅₀ for reducing the human neutrophil activity (based on the data in Fig. 2). Means SEM (n = 4-5 separate experiments). *p < 0.05 decreased IC₅₀ compared to CGS21680.

JMR193 and DWH-146e decreased the stimulated-neutrophil oxidative burst with EC₅₀'s less than 1 nM (0.8 and 0.3 nM, respectively). In contrast, the free acid A_{2A} adenosine receptor agonists DWH-146a and CGS21680 were not as effective in inhibiting the oxidative burst (53 and 9 nM, respectively; Fig. 2). DWH-146e inhibition of the stimulated neutrophil oxidative burst was antagonized by the selective A_{2A} AR antagonist ZM241385.

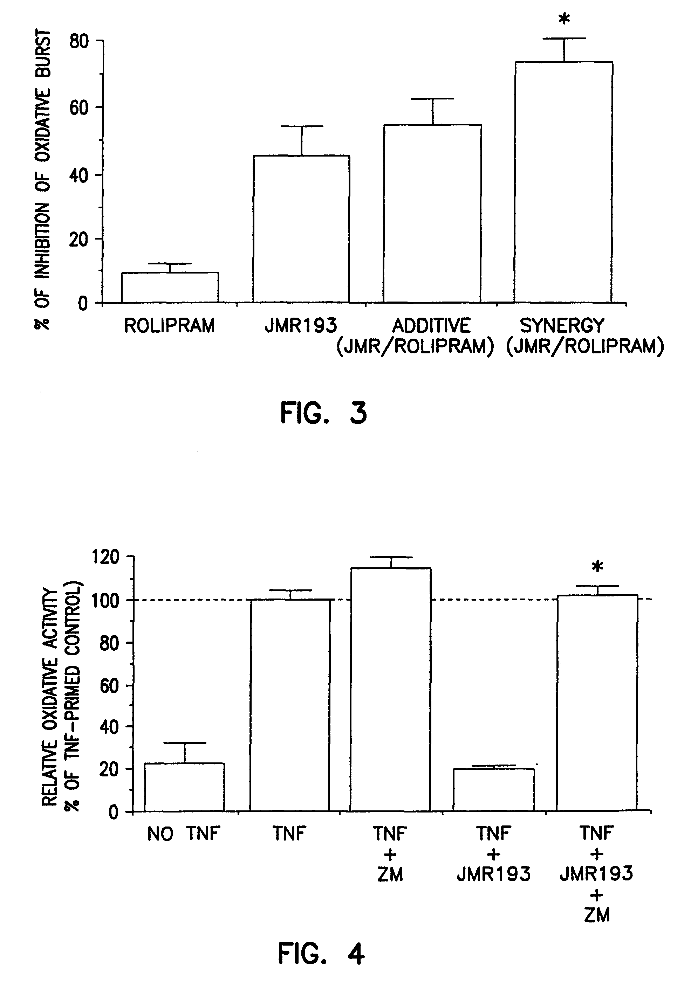

As shown in Fig. 3, JMR193 (1 nM) with rolipram (100 nM) synergistically decreased stimulated release of reactive oxygen species. Human neutrophils were primed with tumor necrosis factor-alpha (1 U/ml) and stimulated with f-met-leu-phe (1 µM). The vertical axis gives the percent inhibition of oxidative activity as measured by luminol-enhanced chemiluminescence. Means SEM (n = 4 separate experiments. *p < 0.05 synergy between JMR193 and rolipram compared to additive activity.

As shown in Fig. 4, the highly selective A_{2A} adenosine receptor antagonist ZM241385 (100 nM) (ZM) counteracted human neutrophil oxidative activity inhibited by JMR193 (10 nM) as measured by luminol-enhanced chemiluminescence. Means SEM of 4 separate experiments. *p = .0004 ZM241385 counteracted JMR193 inhibited oxidative activity.

### E. Human neutrophil [cAMP]ᵢ and neutrophil adherence to a biological surface

A 24 well tissue culture plate was coated with human fibrinogen (5 mg/ml in 1.5% sodium bicarbonate; 0.5 ml/well; Sigma Chemical) overnight at 37°C in 5% CO₂. Neutrophils (3-4 × 10⁶/0.5 ml/sample) were incubated within a well of the coated plate for 45 minutes in 0.5 ml of HBSS containing 0.1% HSA and ADA (1 U/ml) in the presence and absence of recombinant human TNFα (10 U/ml), DWH-146e (3-300 nM), rolipram (300 nM), and/or ZM241385 (100 nM). Following incubation, 0.5 ml HCl (0.2 N) was added to the wells and incubated for 45 minutes more at room temperature to extract the cAMP. The samples were then centrifuged in a microfuge for 2 minutes to remove cell debris. Half ml samples were frozen for cAMP analyses (B. Brooker et al., Science, 194, 270 (1976)). The wells were washed twice with normal saline and the remaining monolayer digested with 0.2 ml of 0.2 N NaOH containing SDS for 2 hours at room temperature. The protein samples were then frozen (-70°C) for later protein analysis to determine relative PMN adherence (K. P. Stowell et al., Anal. Biochem., 85, 572 (1978)).

### Results

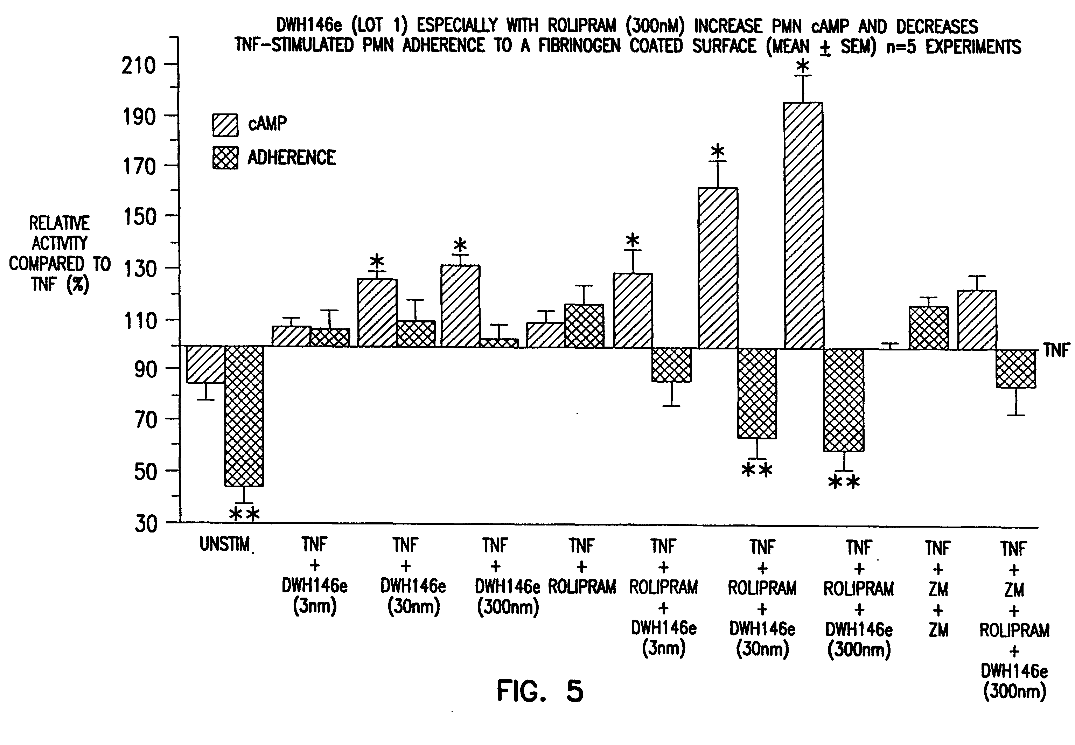

DWH-146e (30-300 nM) alone and synergistically with rolipram (300 nM) increased human neutrophil cAMP content and with rolipram synergistically decreased neutrophil adherence to a fibrinogen-coated surface (Fig. 5). The effects of DWH-146e (300 nM) + rolipram (300 nM) on neutrophil cAMP production and adherence were counteracted by the selective A_{2A} adenosine receptor antagonist, ZM241385 (ZM; 100 nM). Mean SEM of 5 separate experiments. *p < 0.05 increased neutrophil [cAMP] compared to without DWH-146e; **p < 0.05 decreased neutrophil adherence compared to no DWH-146e.

### F. Adherent human neutrophil oxidative activity

Methods. Using methods modified from Section E, neutrophils (2 × 10⁶/ml) from Ficoll-Hypaque separation were incubated 15 minutes 37°C in 0.45 ml of Hanks balanced salt solution containing 0.1% human serum albumin and adenosine deaminase (1U/ml), rolipram (300 nM), and DWH-146e (3-300 nM). Following incubation, cytochrome C (120 µM) and catalase (0.062 mg/ml) are added in the presence and absence of recombinant human tumor necrosis factor-alpha (1U/ml) and 200 µl aliquots of cell suspension were immediately transferred to duplicate wells of a 96 well flat-bottomed tissue culture plate which had been coated overnight with human fibrinogen. The optical density of the samples were read at 550 nm against matched superoxide dismutase (200 U/ml) samples.

### G. Results

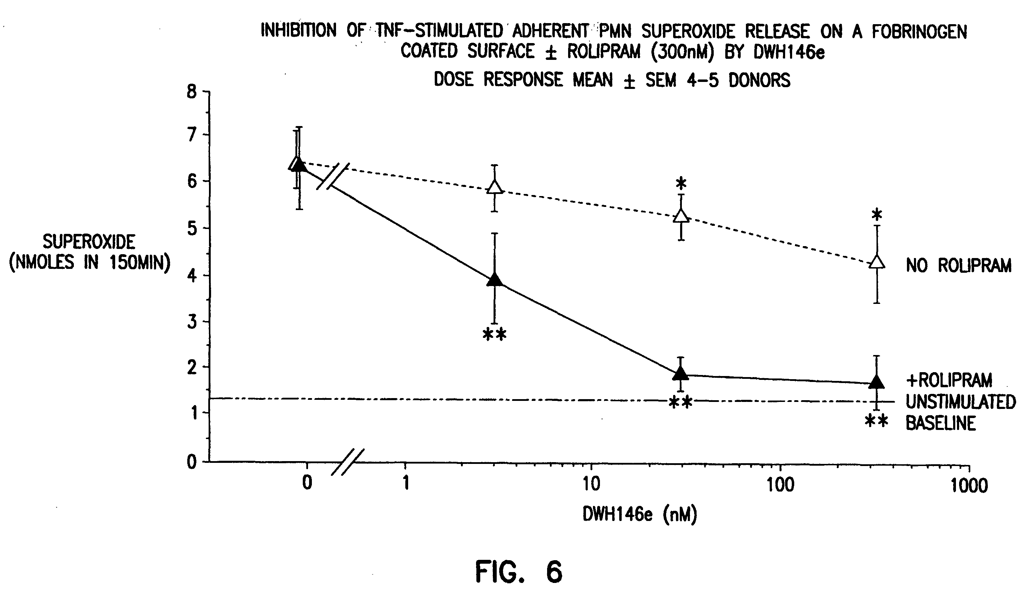

As shown in Fig. 6, inhibition of tumor necrosis factor-alpha (TNF)-stimulated adherent human neutrophil superoxide release on a fibrinogen-coated surface was accomplished by rolipram (300 nM) and DWH-146e. DWH-146e decreased the oxidative burst of adhering neutrophils, and synergistically decreased the oxidative burst in the presence of rolipram, which by itself did not affect neutrophil oxidative activity. The horizontal axis gives the DWH-146e concentration in nM and the vertical axis gives the amount of superoxide released by the neutrophils as measured by cytochrome c reduction. There was marked synergy with DWH-146e and the type IV PDE inhibitor, rolipram, to decrease tumor necrosis factor-alpha-stimulated adherent human neutrophil oxidative activity. Means SEM of replicates from 4-5 separate experiments. *p < 0.05 decreased superoxide release compared to without DWH-146e; **p < 0.05 decreased superoxide release compared to with rolipram and without DWH-146e.

### EXAMPLE 18

### Treatment of Ischemia/Reperfusion (I/R) Injury in Kidney with DWH-146e

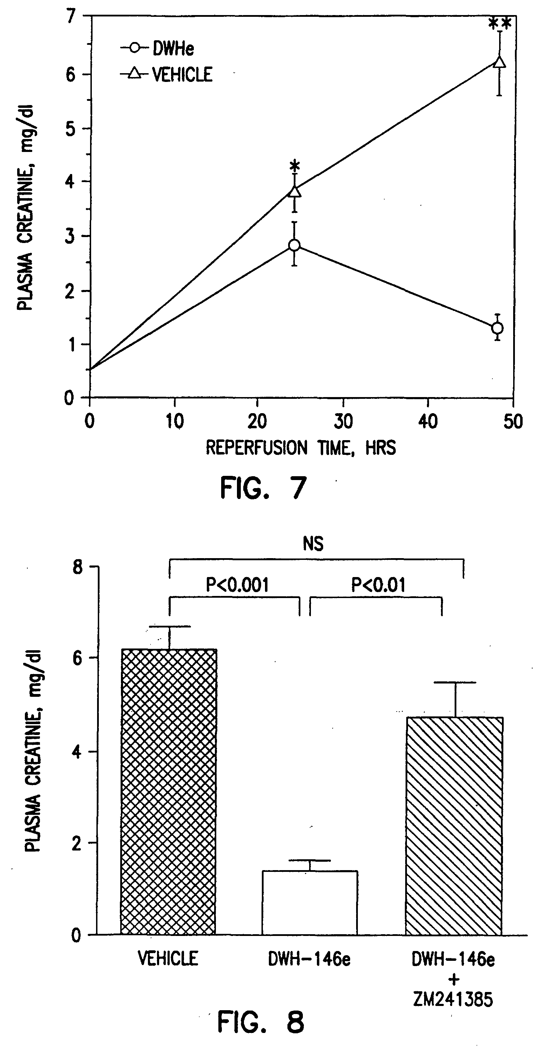

To determine whether or not DWH-146e induced A_{2A} adenosine receptor activation reduces plasma creatinine at 24 and 48 hours following I/R injury in rats, rat kidneys were subjected to 45 minutes ischemia and 24 or 48 hours of reperfusion. DWH-146e (0.004 µg/kg/min) or vehicle was administered continuously via minipump beginning 5 hours prior to I/R. As shown in Fig. 7, DWH-146e significantly decreased plasma creatinine in 7/7 rats (P < 0.05) and in 6/6 rats treated with DWH-146e (P < 0.001), at 24 and 48 hours, respectively.

To determine whether or not the effect of DWH-146e on reduction of plasma creatinine in rats subjected to I/R is A_{2A}-receptor mediated, rat kidneys were subjected to 45 minutes ischemia followed by 48 hours reperfusion. DWH-146e (0.004 µg/kg/min) was administered continuously via minipump beginning 5 hours prior ischemia. As shown in Fig. 8, the improvement in renal function was reversed by the A_{2A} antagonist ZM-241385 (0.003 µg/kg/min-equimolar delivery rate compared with DWH-146e) (*P < 0.001 for Vehicle vs. DWH; **P < 0.05 DWH vs. DWH/ZM. N = 5 for Vehicle, DW; N = 6 for DWH/ZM. ANOVA followed by Bonferroni correction).

DWH-146e, at concentrations that have no hemodynamic effects, prevents renal edema, necrosis and red cell pooling in the inner medulla.

The protection against renal damage afforded by DWH-146e (0.01 µg/kg/min s.c. for 48 hours) was correlated with a dramatic inhibition of neutrophil adherence to vascular endothelium. It is believed that inhibition by DWH-146e of the interaction between neutrophils and vascular endothelium is responsible, at least in part, for the protection against renal damage.

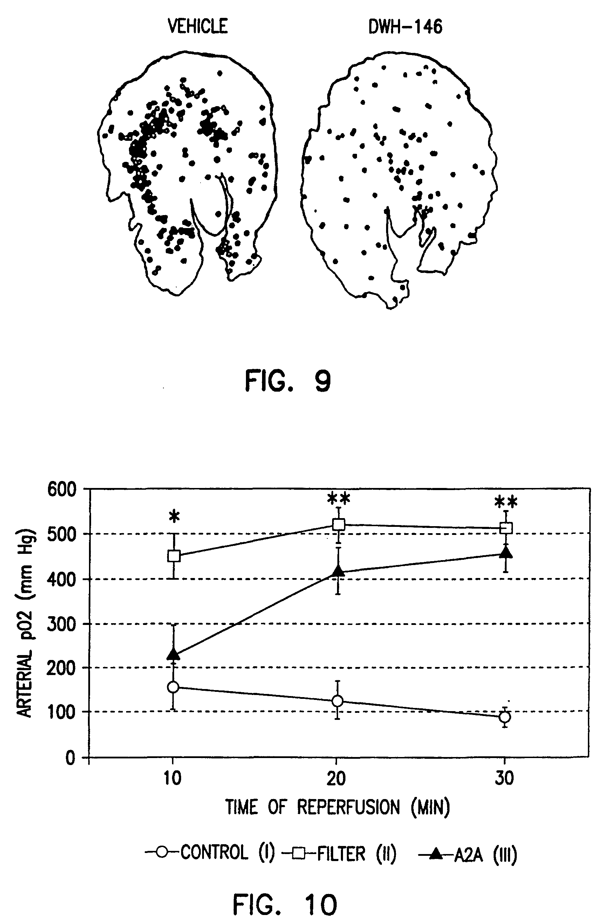

To determine whether or not A_{2A}-AR activation reduces neutrophils in the outer medulla of rats subjected to I/R, using Neurolucida®, the kidney was viewed under 100 × mag and the entire kidney was drawn. PMNs were counted by viewing kidney sections under 250 × mag. Kidney sections were overlaid with optical frames viewed under the microscope and all PMNs were counted within each frame. This system prevents counting of PMNs more than once. As shown in Fig. 9, the density of neutrophils was 15.65/mm² for vehicle and 3.02/mm² for DWH-146e treatment.

### Example 19. Effect of DWH-146e on Lung Reperfusion Injury.

A. Methods. An isolated, whole blood-perfused, ventilated rabbit lung model was used. Donor rabbits underwent lung harvest after pulmonary arterial PGE₁ injection and Euro-Collins preservation solution flush, and lungs were preserved for 18 hours at 4°C. Group I lungs (n=9) served as control subjects. Group II lungs (n=9) were reperfused with whole blood that was first passed through a leukocyte-depleting filter. In group III (n=9), DWH-146e was added to the blood reperfusate (25µg/kg) immediately before reperfusion and was administered throughout the reperfusion period (1 µg/kg/min). All lungs were reperfused for 30 minutes, and pulmonary artery pressure (PAP), pulmonary vascular resistance (PVR), airway compliance (CPL) and arterial oxygenation were recorded. Mycloperoxidase activity (MPO) was recorded to quantify neutrophil sequestration, and wet/dry weight ratios were measured to demonstrate pulmonary edema.

B. Results. Arterial oxygentation in group II and group III was significantly higher than that of group I after 30 minutes of reperfusion (514.27 ± 35.80 and 461.12 ± 43.77 vs. 91.41 ± 20.58 mm Hg, *p*<0.001. As shown in Fig. 10, group III lungs displayed a progressive involvement in pO₂ throughout reperfusion. Leukocyte depletion in group II lungs improved arterial oxygenation in early reperfusion. **p*=0.004 (group II versus groups I and III); ***p*<0.001 (groups II and III versus group I).

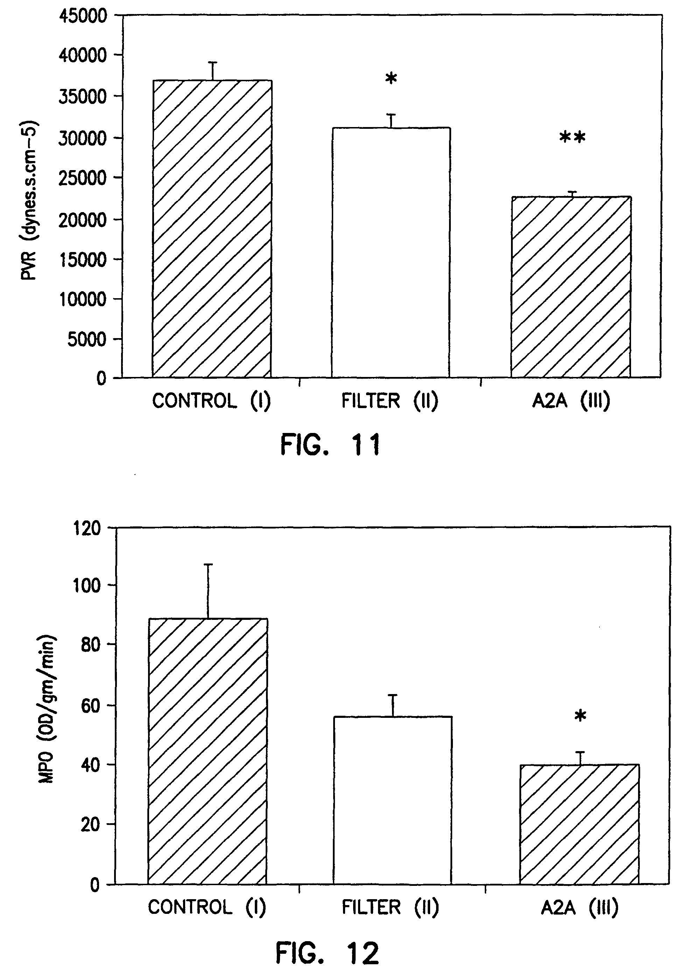

As shown in Fig. 11, mean PVR in group II was significantly reduced when compared to controlled lungs (**p*<0.001). PVR of group III lungs was significantly lower than even those lungs that underwent reperfusion with leukocyte-depleted blood (***p*<0.001 versus groups I and II). Pulmonary vascular resistance was significantly reduced in group III (22,783 ± 357 dynes·s·cm⁻⁵) compared to both group II and group I (31,057 ± 1743 and 36,911 ± 2173 dynes·s·cm⁻⁵, *p*<0.001). Airway compliance was improved in groups II and III when compared to group I (1.68 ± 0.08 and 1.68 ± 0.05 vs. 1.36 ± 0.13, *p*=0.03). Microvascular permeability in group III was reduced to 106.82 ± 17.09 compared with 165.70 ± 21.83 ng Evans-blue dye/gm tissue in group I (*p*=0.05). As shown in Fig 12, myeloperoxidase activity in group III was significantly lower than in group I (**p*=0.03). MPO=myeloperoxidase. Group III myeloperoxidase activity was 39.88 ± 4.87 compared with 88.70 ± 18.69 ΔOD/gm/min in group I (*p*=0.03), and group II myeloperoxidase activity was 56.06 ± 7.46.

C. Conclusions. DWH-146e reduced lung neutrophil sequestration and dramatically improved pulmonary graft function. Neutrophils are important components of the inflammatory cascade of reperfusion injury and their source may include both the circulating blood and the lung graft itself. Selective adenosine-A_{2A} activation interrupts the neutrophil-mediated inflammatory response and reduces lung reperfusion injury following transplantation.

Under light microscopy, control lungs in group I showed severe leukocyte infiltration and edema formation in the alveolar spaces after 18 hours of ischemic storage and 30 minutes of reperfusion. In group II, lungs that underwent reperfusion with leukocyte-depleted blood and in group III lungs (that received DWH-146e during reperfusion, this infiltration was much less.

**Example 20. Effect of DWH-146e on Neointimal Formation after Arterial Injury**. Leukocyte activation with release of inflammatory cytokines occurs after percutaneous coronary intervention and may play a role in restenosis. In the mouse, robust neointima formation in the presence of an intact endothelial lining occurs after ligation of the common carotid artery. Using this model, C57/BL6 mice were randomized at the time of carotid ligation to a 7 day infusion via osmotic pump of DWH-146e, (n=7), or vehicle (n=8).

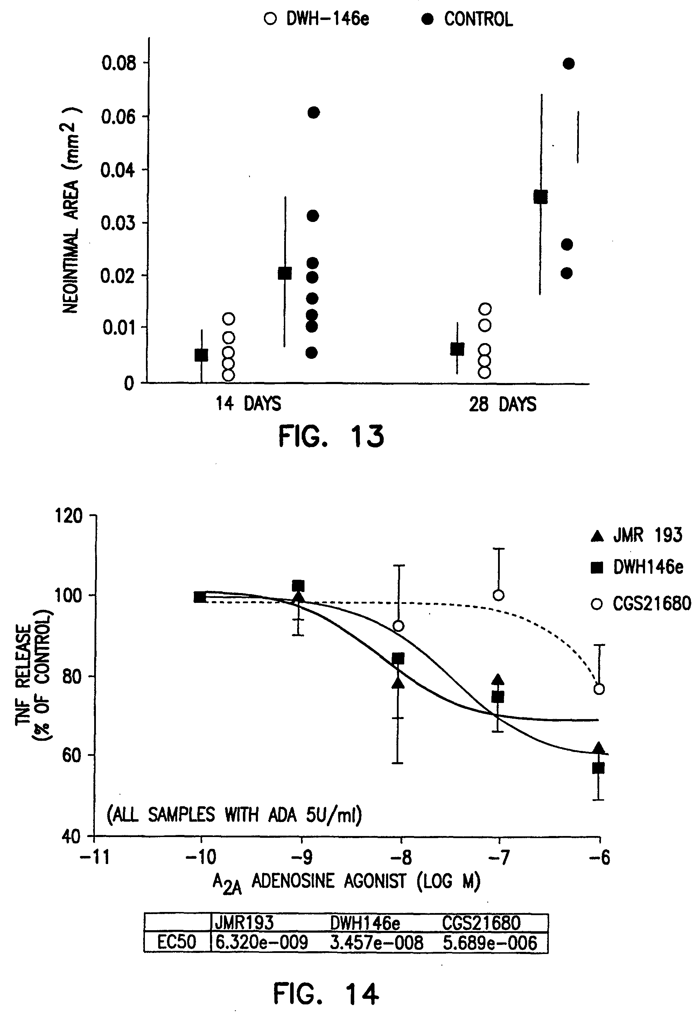

At 14 days after carotid ligation, histomorphometry demonstrated a significant reduction in neointimal area (0.005 ± 0.004 mm² vs. 0.021 ± 0.014 mm², *p*=0.02) and neointimal to medial area ratio (0.13 ± 0.07 vs. 0.64 ± 0.44, *p*=0.01) in the treated animals compared to controls. Medial area was similar in the two groups (0.034 ± 0.007 mm² vs. 0.036 ± 0.009 mm², *p*=0.81). This benefit in limiting neointimal growth persisted to 28 days. Figure 13 summarizes the effect of DWH-146e to inhibit neointimal growth in the mouse LCCA model. These experiments demonstrate that, in a mouse carotid artery ligation model, prolonged A_{2A} stimulation (7 days) by DWH-146e results in a significant reduction in neointimal formation for at least 21 days, possibly through its effect on leukocyte activation and function.

### Example 21. Inhibition of Endotoxin-stimulated Human Monocyte TNFα Release.

### A. Materials.

Ficoll-hypaque was purchased from ICN (Aurora, OH) and Cardinal Scientific (Santa Fe, NM) and Accurate Chemicals and Scientific (Westbury, NY). Endotoxin (lipopolysaccharide; *E. coli* 0111B4) was from List Biologicals (Campbell, CA). Hanks balanced salt solution (HBSS), and limulus amebocyte lysate assay kit were from BioWittaker (Walkersville, MD). Human serum albumin (HSA) was from Cutter Biological (Elkhart, IN). ZM241385 (4-(2-[7-amino-2-(2-furyl)[1,2,4]-triazolo[2,3-a][1,3,5]triazin-5-yl amino]ethyl)phenol) was a gift from Simon Poucher, Zeneca Pharmaceuticals, Cheshire, UK. Stock solutions (1 mM and 10 mM in DMSO) were made and stored at -20°C.

### B. Production of TNFα by purified human adherent monocytes.

Methods: A monocyte rich monolayer (>65% monocytes) was prepared by incubating 1 ml of the mononuclear leukocyte fraction (5 × 10⁵/ml) from a Ficoll-Hypaque separation (A. Ferrante et al., J. Immunol. Meth., 36, 109 (1980)) in wells of a 24 well tissue culture plate (1 hr; 37°C; 5% CO₂). The non-adherent leukocytes were removed by washing and culture medium (1 ml Hanks balanced salt solution, containing 0.1% human serum albumin, adenosine deaminase [5 U/ml] and 1% heat-inactivated autologous serum) added to the wells containing the adherent mononuclear cells. As stated, the following were added: (1) endotoxin (100 ng/ml) and the A_{2A} AR selective antagonist ZM241385 (100 nM) and, (2) A_{2A} adenosine receptor selective agonists JMR193 (1-1000 nM), DWH146e (1-1000 nM) and CGS21680 (10-1000 nM). The samples were then incubated for 4 hours (37°C; 5% CO₂) and the supernatants harvested. Any suspended cells were removed by centrifugation and the cell-free samples frozen (-70°C). TNFα was assayed in the cell-free supernatants (n=6) by an ELISA kit (Coulter/Immunotech, Miami, FL).

### C. Results.

As shown in Figure 10, 14, the A_{2A} adenosine receptor agonists decreased endotoxin-stimulated adherent monocyte production of TNFα. The A_{2A} AR selective antagonist ZM241385 (100 nM) antagonized the effects of JMR193 on TNFα production (Figure 15).

Thus, DWH146e and JMR193 decrease LPS endotoxin-stimulated TNFα production by human monocytes by a mechanism that is dependent upon agonist binding to A_{2A} adenosine receptors.

### EXAMPLE 22. Activity of DWH-146e in Murine Peritonitis Model.

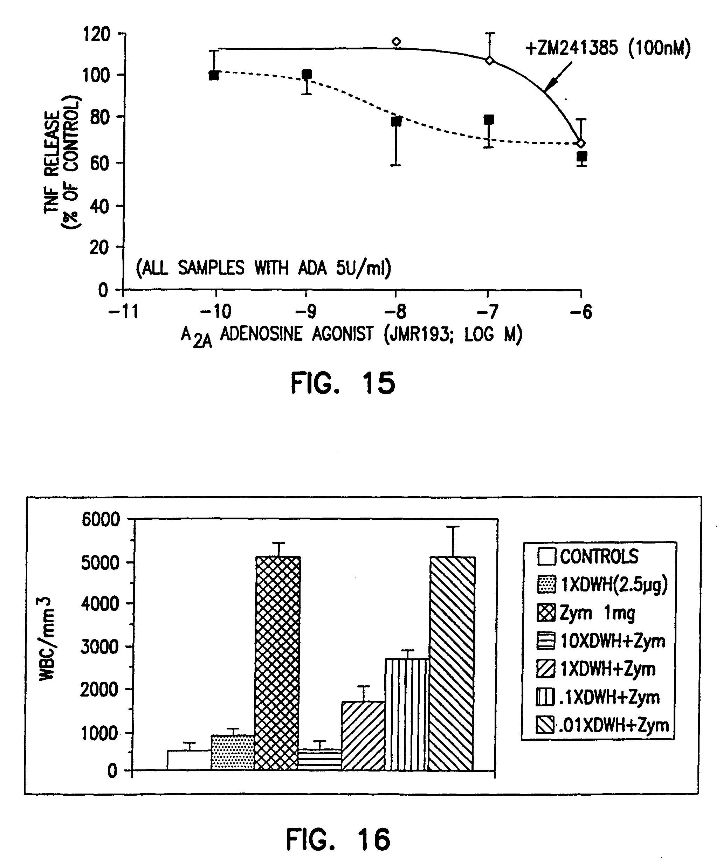

Preliminary experiments with experimental peritonitis have involved the injection of zymosan (Zym) as a potent stimulus of inflammation (Y. Zhang et al., Eur. J. Pharmacol., 313, 237 (1996)). As shown in Figure 16, following injection of zymosan, the mean leukocyte concentration as determined in a neubauer hemocytometer was 7,325 ± 1,893/mm³. Intraperitoneal injection of DWH-146e at a dosage of 2.5 µg/kg one hour prior to zymosan inhibited the development of peritonitis with a mean ± SEM leukocyte concentration of 2,012 ± 374/mm³ 6 hours later (p < 0.05). Thus, these studies demonstrate that the A_{2A} AR is instrumental in mediating PMN traversal into the peritoneum following zymosan challenge.

### Example 23. Cardioprotection Mediated by the Anti-inflammatory Effect of JMR193

The compounds of the invention were tested by inducing myocardial stunning, a form of cardiac injury that occurs following repetitive, transient periods of interrupted coronary blood flow, by repeated occlusion of the blood supply of a coronary artery.

### A. Effect of four cycles of occlusion-reperfusion.

The left anterior descending (LAD) coronary artery of a group of dogs was isolated and encircled with a snare occluder. The dogs LAD artery blood supply was occluded 4 times, for 5 minutes. Following each occlusion blood flow was restored for ten minutes. One group of six dogs were infused with a solution containing the acetate compound (JMR193), prepared in Example 15 (JMR193), (0.01 µg/kg/min) after each occlusion period. A second group of six dogs were infused with a solution containing the vehicle (carrier). After the last occlusion-reperfusion cycle the animals cardiac function was monitored for 3 hours.

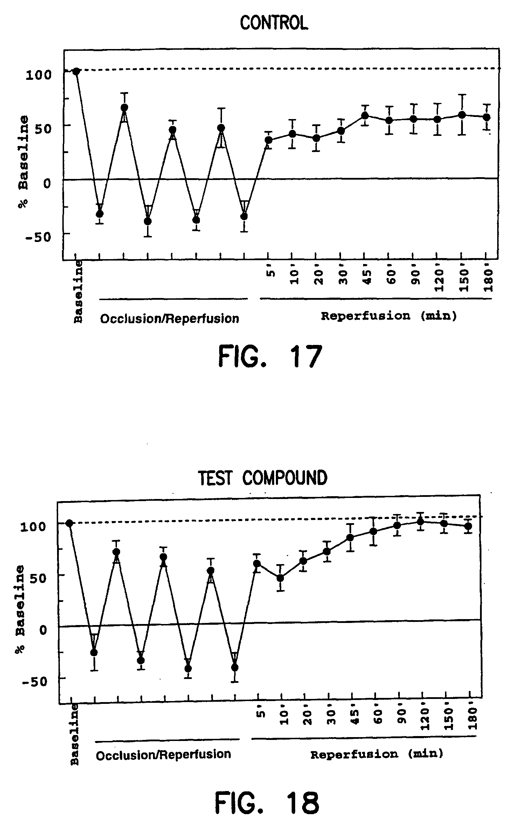

The results are illustrated in Figures 17 and 18. Figure 17 shows the systolic left ventricular (LV) thickening response in 6 control dogs. Cardiac thickening was reduced by approximately 50% 3 hours after the last occlusion. Figure 18 shows the LV thickening response in the 6 dogs that received an i.v. infusion of the test compound, JMR193 (0.01 µg/kg/min), beginning during the baseline period and continuing throughout the experiment. The cardiac function, with JMR193 infusion, returned to nearly normal as early as 90 min post reperfusion.

### B. Effect of ten cycles of occlusion-reperfusion.

Two additional groups of dogs were subjected to ten (rather than 4) occlusion-reperfusion cycles, where the each occlusion was 5 minutes, interspersed by 5 minutes of reperfusion. In this example, two of the animals were infused with a solution containing the acetate compound (JMR193), prepared in Example 15, (0.01 µg/kg/min) after each occlusion period. Another three animals were infused with a solution containing the vehicle (carrier). After the last occlusion-reperfusion cycle the animals cardiac function was monitored for 3 hours.

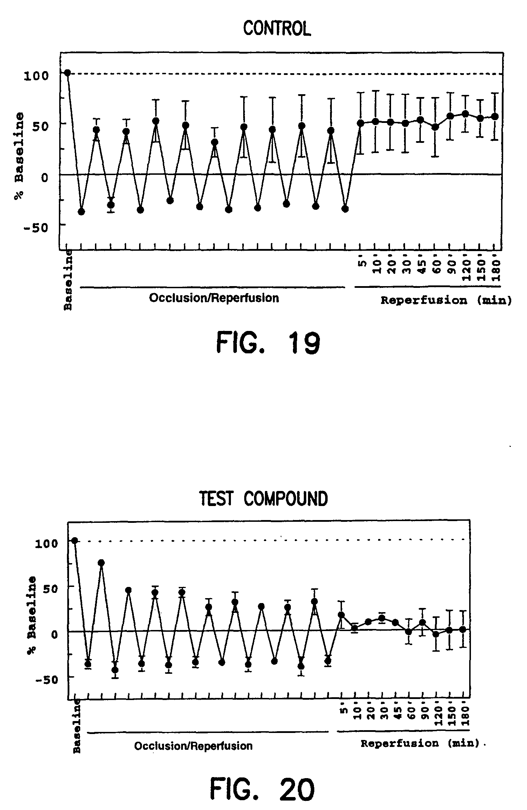

The results are illustrated in Figures 19 and 20. Figure 19 shows the systolic left ventricular thickening response in the 3 control dogs. This was a more severe cardiac insult, than in Example 23A, and as a result the LV thickening was completely absent early after reperfusion and remained akinetic for 3 hours. Figure 20 shows the LV thickening in the 2 dogs that received an i.v. infusion of the test compound, JMR193 (0.01 µg/kg/min) beginning during the baseline period and continuing throughout the experiment. Compared with the control group, the dogs that received the JMR193 infusion showed a significant and marked improvement in cardiac function immediately after reperfusion which persisted for 3 hours.

### C. Effect of acetate compound JMR193 on uptake of neutrophils during occlusion-reperfusion.

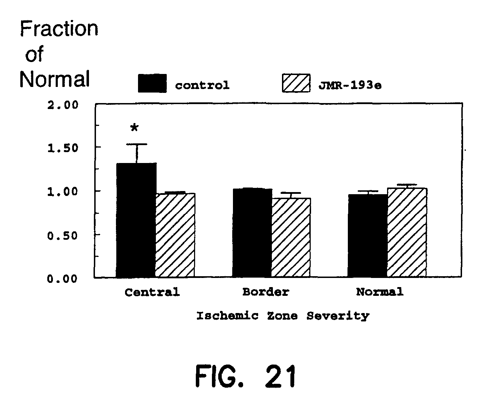

Some animals were administered radiolabeled neutrophils. (Neutrophils were isolated from dog blood, incubated with a compound containing ^{99m}Tc, and reinjected into the dogs.) The ^{99m}Tc- labeled neutrophils were administered intravenously as a marker to determine the level of inflammation in the reperfusion zone, following four ischemic-reperfusion cycles. The inflammation from the occlusion-reperfusion cycles caused adherence of the radioactive neutrophils and was quantified with a gamma-camera. The neutrophil adherence was inhibited by the JMR193. The results are illustrated in Figure 21 where the localization of ^{99m}Tc-labeled neutrophils in the dogs treated with vehicle alone (solid bars) is greater than the JMR193 treated (striped bars) dogs. Thus, the reduction of radiolabeled neutrophils in the central ischemic zone caused by the JMR193 infusion illustrates the reduction of (*) cardiac inflammation.

The studies described in Examples 23A and 23B indicate that cardiac inflammation plays a significant injurious role in causing myocardial stunning. In addition, the administration of an adenosine A_{2A} receptor agonist such as, for example, JMR-193 either prevents mild stunning (Figures 17 and 18) or significantly attenuates myocardial dysfunction accompanying severe stunning (Figures 19 and 20).

## Claims

1. A compound of the formula (I): 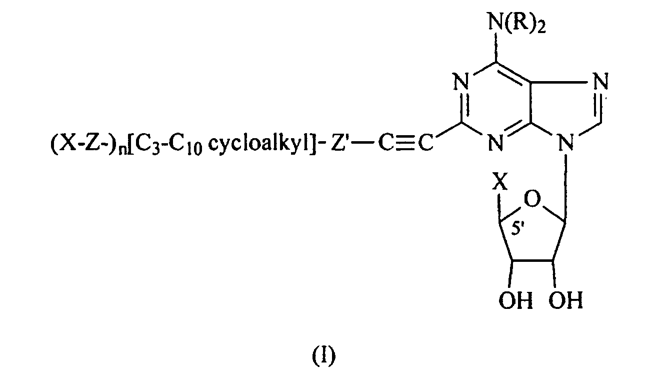 wherein
(a) each R is individually hydrogen, C₁-C₆ alkyl, C₃-C₇ cycloalkyl, phenyl or phenyl(C₁-C₃)-alkyl;
(b) X is -CH₂OH, -CO₂R², -OC(O)R², or C(O)NR³R⁴;
(c) each of R², R³ and R⁴ is individually H, C₁₋₆-alkyl; C₁₋₆-alkyl substituted with 1-3 C₁₋₆-alkoxy, C₃-C₇ cycloalkyl, C₁₋₆-alkylthio, halogen, hydroxy, amino, mono(C₁₋₆-alkyl)amino, di(C₁₋₆-alkyl)amino, or C₆₋₁₀-aryl, wherein aryl may be substituted with 1-3 halogen, C₁₋₆-alkyl, hydroxy, amino, mono(C₁₋₆-alkyl)amino, or di(C₁₋₆-alkyl)amino; C₆₋₁₀-aryl; or C₆₋₁₀-aryl substituted with 1-3 halogen, hydroxy, amino, mono(C₁₋₆-alkyl)amino, di(C₁₋₆-alkyl)amino or C₁₋₆-alkyl;
(d) Z and Z' are individually (C₁-C₆)alkyl, optionally interrupted by 1-3 S or non-peroxide O, or are absent, and n is 1-3; or a pharmaceutically acceptable salt thereof.

2. A compound of claim 1 wherein 5'-X is -CH₂OH or -C(O)NR³R⁴.

3. The compound of claim 2 wherein 5'-X is -C(O)NR³R⁴.

4. A compound of claim 3 wherein R³ is H and R⁴ is (C₁-C₄)alkyl.

5. A compound of claim 1 wherein each R is H or (C₁-C₄)alkyl.

6. A compound of claim 1 wherein Z' is -CH₂- or -CH₂-CH₂-.

7. A compound of claim 6 wherein Z is -CH₂- or -CH₂-CH₂-.

8. A compound of claim 1 wherein C₃-C₁₀ cycloalkyl is cyclohexyl or cyclopentyl.

9. A compound of claim 8 wherein X is (C₁-C₄)alkoxycarbonyl, C(O)NR³R⁴ or acetoxymethyl.

10. A compound of claim 8 wherein X-Z is HO₂C-Z-.

11. A compound of claim 8 wherein X-Z and Z' are *trans* on C₃-C₁₀ cycloalkyl.

12. A compound of claim 1 wherein R is H, 5'-X is ethylaminocarbonyl, and (X-Z-)ₙ[(C₃-C₁₀)-cycloalkyl]-Z'-C≡C- is 2-(4-methoxycarbonylcyclohexylmethyl)ethynyl or 2-(4-carboxy-cyclohexylmethyl)ethynyl.

13. A compound of claim 1 wherein R is H, 5'-X is ethylaminocarbonyl, and (X-Z-)ₙ[(C₃-C₁₀)-cycloalkyl]-Z'-C≡C- is 2-(4-acetoxymethylcyclohexylmethyl)ethynyl.

14. [4-(3-{9-(2R,3R,4S,5S)-5-(N-Ethylcarbamoyl)-3,4-dihydroxyoxolan-2-yl]-6-aminopurin-2-yl}prop-2-ynyl)cyclohexyl]methyl acetate or a pharmaceutically acceptable salt thereof.

15. [(2R,3R,4S,5S)-5-(6-amino-2-{3-[4-(hydroxymethyl)cyclohexyl]prop-1-ynyl}purin-9-yl)-3,4-dihydroxyoxolan-2-yl]-*N*-ethylcarboxamide or a pharmaceutically acceptable salt thereof.

16. Methyl 4-(3-{9-[(2R,3R,4S,5S)-5-(N-ethylcarbamoyl)-3,4-dihydroxyoxolan-2-yl]-6-aminopurin-2-yl)}prop-1-ynyl)cyclohexane-carboxylate or a pharmaceutically acceptable salt thereof.

17. 4-(3-{9-[(2R,3R,4S,5S)-5-(N-Ethylcarbamoyl)-3,4-dihydroxyoxolan-2-yl]-6-aminopurin-2-yl)}prop-1-ynyl)cyclohexane carboxylic acid or a pharmaceutically acceptable salt thereof.

18. A compound of any of claims 1 to 17, for use in medical therapy.

19. A compound of claim 18, wherein the medical therapy is inhibition of an inflammatory response.

20. The compound of claim 18 wherein the medical therapy further comprises the use of a Type IV phosphodiesterase inhibitor.

21. The compound of claim 20 wherein the inhibitor is rolipram.

22. The compound of claim 19 wherein the inflammatory response is due to ischemia, atherosclerosis, an autoimmune disease, ischemia/reperfusion injury, stroke, traumatic brain injury, spinal cord injury, organ transplantation, tissue transplantation, cell transplantation, a skin disease, infection, angioplasty, stent placement, shunt placement or grafting, an allergic disease; a wasting disease, immunosuppressive therapy or a pathological condition or symptom in a mammal, wherein the activity of A_{2A} adenosine receptors is implicated and agonism of such activity is desired.

23. The compound of claim 19 wherein the inflammatory response is at the heart, kidney, or lung.

24. Use of a compound of any of claims 1-17 to prepare a medicament useful for treating an inflammatory response.

25. The use of claim 24 wherein the medicament comprises a Type IV phosphodiesterase inhibitor.

26. The use of claim 25 wherein the phosphodiesterase inhibitor is rolipram.

27. The use of claim 25 wherein the medicament comprises a liquid carrier.

28. The use of claim 25 wherein the medicament is adapted for parenteral administration.

## Patentansprüche

1. Verbindung der Formel (I): 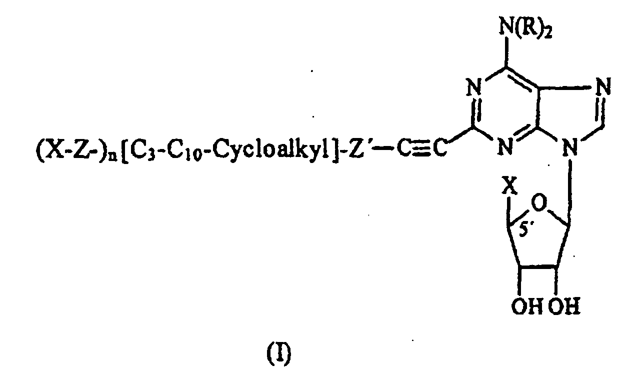 wobei
(a) jeder R individuell ein Wasserstoff, ein C₁-C₆-Alkyl, ein C₃-C₇-Cycloalkyl, ein Phenyl oder ein Phenyl(C₁-C₃)alkyl ist;
(b) X -CH₂OH, -CO₂R², -OC(O)R² oder C(O)NR³R⁴ ist;
(c) jeder von R², R³ und R⁴ individuell ein H, ein C₁₋₆-Alkyl; ein C₁₋₆-Alkyl, substituiert mit 1-3 C₁₋₆-Alkoxy, C₃₋₇-Cycloalkyl, C₁₋₆-Alkylthio, Halogen, Hydroxy, Amino, Mono(C₁₋₆-Alkyl)amino, Di(C₁₋₆-Alkyl)amino, oder C₆₋₁₀-Aryl, wobei das Aryl mit 1-3 Halogen, C₁₋₆-Alkyl, Hydroxy, Amino, Mono(C₁₋₆-Alkyl)amino oder Di(C₁₋₆-Alkyl)amino substituiert sein kann; ein C₆₋₁₀-Aryl; oder ein C₆₋₁₀-Aryl, substituiert mit 1-3 Halogen, Hydroxy, Amino, Mono(C₁₋₆-Alkyl)amino, Di(C₁₋₆-Alkyl)amino oder C₁₋₆-Alkyl ist;
(d) Z und Z' individuell (C₁-C₆)Alkyl optional unterbrochen von 1-3 S oder einem O, der kein Peroxid ist, sind oder die abwesend sind und n 1-3 ist; oder ein pharmazeutisch akzeptables Salz davon.

2. Verbindung nach Anspruch 1, wobei 5'-X -CH₂OH oder -C(O)NR³R⁴ ist.

3. Verbindung nach Anspruch 2, wobei 5'-X -C(O)NR³R⁴ ist.

4. Verbindung nach Anspruch 3, wobei R³ ein H und R⁴ ein (C₁-C₄)Alkyl ist.

5. Verbindung nach Anspruch 1, wobei jeder R ein H oder ein (C₁-C₄)Alkyl ist.

6. Verbindung nach Anspruch 1, wobei Z' -CH₂- oder -CH₂-CH₂- ist.

7. Verbindung nach Anspruch 6, wobei Z -CH₂- oder -CH₂-CH₂- ist

8. Verbindung nach Anspruch 1, wobei das C₃-C₁₀-Cycloalkyl ein Cyclohexyl oder Cyclopenthyl ist

9. Verbindung nach Anspruch 8, wobei X ein (C₁-C₄)Alkoxycarbonyl, C(O)NR³R⁴ oder ein Acetoxymethyl ist.

10. Verbindung nach Anspruch 8, wobei X-Z HO₂C-Z- ist.

11. Verbindung nach Anspruch 8, wobei X-Z und Z' *trans* an dem C₃-C₁₀-Cycloalkyl stehen.

12. Verbindung nach Anspruch 1, wobei R ein H, 5'-X ein Ethylaminocarbonyl und (X-Z-)ₙ[(C₃-C₁₀)-Cycloalkyl]-Z'-C≡C- 2-(4-Methoxycarbonylcyclohexylmethyl)ethynyl oder 2-(4-Carboxycyclohexylmethyl)ethynyl ist.

13. Verbindung nach Anspruch 1, wobei R ein H, 5'-X ein Ethylaminocarbonyl und (X-Z-)ₙ[(C₃-C₁₀)-Cycloalkyl]-Z'-C≡C- 2-(4-Acetoxymethylcyclohexylmethyl)ethynyl ist.

14. [4- (3- {9-(2R, 3R, 4S, 5S)-5-(N-Ethylcarbamoyl)-3,4-dihydroxyoxolan-2-yl]-6-aminopurin-2-yl}prop-2-ynyl)cyclohexyl]methylacetate oder ein pharmazeutisch akzeptables Salz davon.

15. [(2R, 3R, 4S, 5S)-5-(6-Amino-2-{3-[4-(hydroxymethyl)cyclohexyl] prop-1-ynyl}purin-9-yl)-3,4-dihydroxyoxolan-2-yl]-*N*-ethylcarboxamide oder ein pharmazeutisch akzeptables Salz davon.

16. Methyl-4-(3-{9-[(2R, 3R, 4S, 5S)-5-(N-ethylcarbamoyl)-3,4-dihydroxyoxolan-2-yl]-6-aminopurin-2-yl)}prop-1-ynyl)cyclohexancarboxylat oder ein pharmazeutisch akzeptables Salz davon.

17. 4-(3-{9-[(2R, 3R, 4S, 5S)-5-(N-Ethylcarbamoyl)-3,4-dihydroxyoxolan-2-yl]-6-aminopurin-2-yl)}prop-1-ynyl)cyclohexancarbonsäure oder ein pharmazeutisch akzeptables Salz davon.

18. Verbindung nach einem der Ansprüche 1 bis 17 für die Verwendung in der medizinischen Therapie.

19. Verbindung nach Anspruch 18, wobei die medizinische Therapie die Inhibierung einer entzündlichen Reaktion ist.

20. Verbindung nach Anspruch 18, wobei die medizinische Therapie darüber hinaus die Verwendung eines Typ IV Phosphodiesterasehemmers umfasst.

21. Verbindung nach Anspruch 20, wobei der Hemmer Rolipram ist.

22. Verbindung nach Anspruch 19, wobei die entzündliche Reaktion auf Grund einer Ischämie, einer Atherosklerose, einer Autoimmunerkrankung, einer Ischämie/Reperfusionsverletzung, eines Schlaganfalls, einer traumatischen Hirnverletzung, einer Rückenmarksverletzung, einer Organtransplantation, einer Gewebstransplantation, einer Zelltransplantation, einer-Hauterltrankung, einer Infektion, einer Angioplastie, der Legung eines Stents, der Legung eines Shunts oder des Graftings eines Shunts, einer allergischen Erkrankung; einer zehrenden Krankheit, einer immunsuppressiven Therapie oder einer pathologischen Kondition oder einem pathologischen Symptom in einem Säugetier verursacht ist, wobei die Aktivität der A_{2A}-Adenosinrezeptoren mit einbezogen ist und der Agonismus einer solchen Aktivität gewünscht ist.

23. Verbindung nach Anspruch 19, wobei die entzündliche Reaktion im Herz, in der Niere oder der Lunge ist.

24. Verwendung der Verbindung nach einem der Ansprüche 1-17, um ein Medikament herzustellen, das für die Behandlung einer entzündlichen Reaktion nützlich ist.

25. Verwendung von Anspruch 24, wobei das Medikament einen Typ IV Phosphodiesterasehemmer umfasst.

26. Verwendung von Anspruch 25, wobei der Phosphodiesterasehemmer Rolipram ist.

27. Verwendung von Anspruch 25, wobei das Medikament einen flüssigen Träger umfasst.

28. Verwendung von Anspruch 25, wobei das Medikament für eine parenterale Verabreichung geeignet ist.

## Revendications

1. Composé de formule (I) : 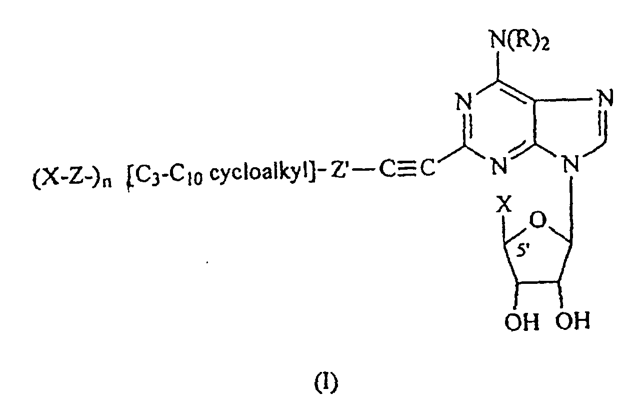 dans laquelle
(a) chaque R représente, individuellement, un atome d'hydrogène, un groupe alkyle en C₁ à C₆, cycloalkyle en C₃ à C₇, phényle ou phényl-(alkyle en C₁ à C₃) ;
(b) X représente -CH₂OH, -CO₂R², -OC(O)R² ou C(O)NR³R⁴ ;
(c) chaque R², R³ et R⁴ représentent, individuellement, H, un groupe alkyle en C₁ à C₆ ; un groupe alkyle en C₁ à C₆ substitué par 1 à 3 groupes alcoxy en C₁ à C₆, cycloalkyle en C₃ à C₇, alkylthio en C₁ à C₆, halogéno, hydroxy, amino, mono(alkyle en C₁ à C₆)amino, di(alkyle en C₁ à C₆)amino ou aryle en C₆ à C₁₀, dans lequel le groupe aryle peut être substitué par 1 à 3 atomes d'halogène, un groupe alkyle en C₁ à C₆, hydroxy, amino, mono(alkyle en C₁ à C₆)amino ou di(alkyle en C₁ à C₆)amino ; un groupe aryle en C₆ à C₁₀ ; ou un groupe aryle en C₆ à C₁₀ substitué par 1 à 3 atomes d'halogène, un groupe hydroxy, amino, mono(alkyle en C₁ à C₆)amino, di(alkyle en C₁ à C₆)amino ou alkyle en C₁ à C₆ ;
(d) Z et Z' représentent, individuellement, un groupe alkyle en C₁ à C₆, éventuellement interrompu par 1 à 3 atomes de S ou O non peroxyde, ou sont absents, et n vaut 1 à 3 ; ou un sel acceptable sur le plan pharmaceutique de ceux-ci.

2. Composé selon la revendication 1, dans lequel 5'-X est -CH₂OH ou -C(O)NR³R⁴.

3. Composé selon la revendication 2, dans lequel 5'-X est -C(O)NR³R⁴.

4. Composé selon la revendication 3, dans lequel R³ représente H et R⁴ représente un groupe alkyle en C₁ à C₄.

5. Composé selon la revendication 1, dans lequel chaque R représente H ou un groupe alkyle en C₁ à C₄.

6. Composé selon la revendication 1, dans lequel Z' représente -CH₂- ou -CH₂-CH₂-.

7. Composé selon la revendication 6, dans lequel Z représente -CH₂- ou -CH₂-CH₂-.

8. Composé selon la revendication 1, dans lequel un groupe cycloalkyle en C₃ à C₁₀ est un groupe cyclohexyle ou cyclopentyle.

9. Composé selon la revendication 8, dans lequel X est un groupe (alcoxy en C₁ à C₄)-carbonyle, C(O)NR³R⁴ ou acétoxyméthyle.

10. Composé selon la revendication 8, dans lequel X-Z représente HO₂C-Z-.

11. Composé selon la revendication 8, dans lequel X-Z et Z' *sont trans sur* un groupe cycloalkyle en C₃ à C₁₀.

12. Composé selon la revendication 1, dans lequel R est H, 5'-X est un groupe éthylaminocarbonyle et (X-Z-)ₙ[cycloalkyle en C₃ à C₁₀]-Z'-C≡C- est le 2-(4-méthoxycarbonyl-cyclohexylméthyl)éthynyle ou le 2-(4-carboxy-cyclohexylméthyl)éthynyle.

13. Composé selon la revendication 1, dans lequel R est H, 5'-X est un groupe éthylaminocarbonyle et (X-Z-)ₙ[cycloalkyle en C₃ à C₁₀]-Z'-C≡C- est le 2-(4-acéthoxyméthyl-cyclohexylméthyl)éthynyle.

14. Acétate de [4-(3-(9-(2R,3R,4S,5S)-5-(N-éthylcarbamoyl)-3,4-dihydroxyoxolan-2-yl]-6-aminopurin-2-yl}prop-2-ynyl)cyclohexyl]méthyle ou un de ses sels acceptables sur le plan pharmaceutique.

15. [(2R,3R,4S,5S)-5-(6-amino-2-{3-[4-(hydroxyméthyl)cyclohexyl]prop-1-ynyl}purin-9-yl)-3,4-dihydroxyoxclan-2-yl]-N-éthylcarboxamide ou un de ses sels acceptables sur le plan pharmaceutique.

16. 4-(3-{9-[(2R,3R,4S,5S)-5-(N-éthylcarbamoyl)-3,4-dihydroxyoxolan-2-yl]-6-aminopurin-2-yl)}prop-1-ynyl)cyclohexane-carboxylate de méthyle ou un de ses sels acceptables sur le plan pharmaceutique.

17. Acide 4-(3-{9-[(2R,3R,4S,5S)-5-(N-éthylcarbamoyl)-3,4-dihydroxyoxolan-2-yl]-6-aminopurin-2-yl)}prop-1-ynyl)cyclohexane-carboxylique ou un de ses sels acceptables sur le plan pharmaceutique.

18. Composé selon l'une quelconque des revendications 1 à 17, pour une utilisation dans un traitement médical.

19. Composé selon la revendication 18, dans lequel le traitement médical est l'inhibition d'une réponse inflammatoire.

20. Composé selon la revendication 18, dans lequel le traitement médical comprend de plus l'utilisation d'un inhibiteur de phosphodiestérase de type IV.

21. Composé selon la revendication 20, dans lequel l'inhibiteur est le rolipram.

22. Composé selon la revendication 19, dans lequel la réponse inflammatoire est due à une ischémie, une athérosclérose, une maladie autoimmune, une lésion ischémie/reperfusion, un accident vasculaire cérébral, une lésion cérébrale traumatique, une lésion de la moelle épinière, une transplantation d'organe, une transplantation de tissu, une transplantation cellulaire, une maladie de la peau, une infection, une angioplastie, la mise en place d'une endoprothèse vasculaire, la mise en place ou la greffe d'un shunt, une maladie allergique; une maladie atrophique, un traitement immunosuppresseur ou un état ou un symptôme pathologique chez un mammifère, dans lequel l'activité des récepteurs d'adénosine A_{2A} est mise en cause et l'agonisme d'une telle activité est souhaité.

23. Composé selon la revendication 19, dans lequel la réponse inflammatoire se situe au coeur, aux reins ou au poumons.

24. Utilisation d'un composé selon l'une quelconque des revendications 1 à 17 pour préparer un médicament utile pour traiter une réponse inflammatoire.

25. Utilisation selon la revendication 24, dans laquelle le médicament comprend un inhibiteur de phosphodiestérase de type IV.

26. Utilisation selon la revendication 25, dans laquelle l'inhibiteur de phosphodiestérase est le rolipram.

27. Utilisation selon la revendication 25, dans laquelle le médicament comprend un véhicule liquide.

28. Utilisation selon la revendication 25, dans laquelle le médicament est adapté pour une administration parentérale.
